(19) Europäisches Patentamt
European Patent Office
Office européen des brevets

(11) EP 2 576 522 B1

(12) EUROPEAN PATENT SPECIFICATION

(45) Date of publication and mention of the grant of the patent:
**05.11.2014 Bulletin 2014/45**

(21) Application number: **11730409.7**

(22) Date of filing: **21.03.2011**

(51) Int Cl.:
***C07D 257/02** (2006.01)*

(86) International application number:
**PCT/IB2011/000585**

(87) International publication number:
**WO 2011/151683 (08.12.2011 Gazette 2011/49)**

(54) **ZN (II) BASED COLORIMETRIC SENSOR AND PROCESS FOR THE PREPARATION THEREOF**

KOLORIMETRISCHER SENSOR AUF ZN (II)-BASIS UND HERSTELLUNGSVERFAHREN DAFÜR

CAPTEUR COLORIMÉTRIQUE À BASE DE ZN(II) ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2010 IN DE12422010**

(43) Date of publication of application:
**10.04.2013 Bulletin 2013/15**

(73) Proprietor: **Council of Scientific & Industrial Research**
**New Delhi 110 001 (IN)**

(72) Inventors:
- **MAHATO, Prasenjit**
  **Gujarat (IN)**
- **GHOSH, Amrita**
  **Gujarat (IN)**
- **MISHRA, Sanjiv, Kumar**
  **Gujarat (IN)**
- **SHRIVASTAVA, Anupama**
  **Gujarat (IN)**
- **MISHRA, Sandhya**
  **Gujarat (IN)**
- **DAS, Amitava**
  **Gujarat (IN)**

(74) Representative: **Goddar, Heinz J.**
**Boehmert & Boehmert**
**Anwaltspartnerschaft mbB**
**Patentanwälte Rechtsanwälte**
**Pettenkoferstrasse 20-22**
**80336 München (DE)**

(56) References cited:
- **JOSE D AMILAN ET AL: "Colorimetric sensor for ATP in aqueous solution.", ORGANIC LETTERS 10 MAY 2007 LNKD- PUBMED:17429979, vol. 9, no. 10, 10 May 2007 (2007-05-10), pages 1979-1982, XP000002656705, ISSN: 1523-7060 cited in the application**
- **O'NEIL LAUREN L ET AL: "A selective, noncovalent assay for base flipping in DNA", JOURNAL OF THE AMERICAN CHEMICAL SOCIETY, vol. 127, no. 48, December 2005 (2005-12), pages 16800-16801, XP000002656706, ISSN: 0002-7863**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 576 522 B1

## Description

### FIELD OF THE INVENTION

The present invention relates to compound of formula 1A useful as a viable staining agent for living cells.

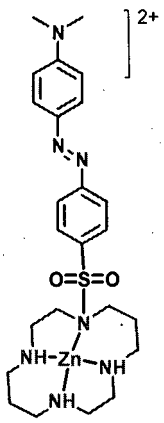

Formula 1A

The present invention also relates to a Zn(II) based colorimetric sensor (tetraaza macrocyclic complex of Zn(II)) having selectivity for adenosine triphosphate (ATP) compared to adenosine monophosphate (AMP), adenosine diphosphate (ADP), pyrophosphate (PPi), cytosine triphosphate (CTP) and phosphate in aqueous and physiological pH.

The present invention also relates to zinc complex **(L.Zn)** (Scheme 1, Formula 1A) useful as staining agent for the Eukaryotic cells as well as for Prokaryotic cells, used to study the cell growth dynamics, used for research in Cystic Fibrosis owing to its solubility in pure water.

### BACKGROUND OF THE INVENTION

Sensor molecules are generally composed of a receptor fragment, specific for a target analyte and covalently bound to a signaling unit, which is capable of reporting the binding induced changes in spectral (electronic spectra, fluorescence spectra and NMR spectra) properties (for chromogenic signaling units) or redox potential data (for redox active signaling unit). Among all these, colorimetric sensors have a definite edge over the other owing to naked eye detection of the binding phenomena. [(a) Vázquez, M.; Fabbrizzi, L.; Taglietti A.; Pedrido, R. M.; González-Noya, A. M.; Bermejo, M. R. Angew. Chem. Int. Ed. 2004, 43, 1962. (b) Lin, Z.; Ou, S.; Duan, C.; Zhang, B.; Bai, Z. Chem. Commun. 2006, 624. (c) Miyaji, H.; Sessler J. L. Angew. Chem. Int. Ed. 2001, 40, 154. (d) Libra, E. R.; Scott, M. J. Chem. Commun. 2006, 1485. (e) Zhang, D.; Zhang, M.; Liu Z.; Yu, M.; Li, F.; Yi, T.; Huang, C. Tetrahedron Lett. 2006, 47, 7093. (f) Han, M. S.; Kim, D.H. Angew. Chem. Int. Ed. 2002, 41, 3809. (g) Jose, D. A.; Kar, P.; Koley, D.; Ganguly, B.; Thiel, W.; Ghosh, H.N.; Das, A. Inorg. Chem., 2007, 46, 5576. (h) Jose, D. A.; Kumar, D. K.; Kar, P.; Verma, S.; Ghosh, A.; Ganguly, B.; Ghosh, H. N.; Das, A. Tetrahedron, 2007, 63, 12007. (i) Jose, D. A.; Kumar, D. K.; Ganguly, B.; Das, A. Org. Lett. 2004, 6, 3445. (j) Jose, D. A.; Kumar, D. K.; Ganguly, B.; Das, A. Tetrahedron Lett. 2005, 46, 5343. (k) Jose,D. A.; Singh, A.; Ganguly, B.; Das, A. Tetrahedron Lett. 2007, 48, 3695 (1) Hu, S.; Guo, Y.; Xu, J.; Shao, S. Org. Biomol. Chem. 2008, 6, 2071.]

It is a generally accepted fact, that fluorescence based sensors allows the detection of very low concentration of the desired analyte. Thus, it is a challenge to the chemists and material scientist to develop a colorimetric sensor that allows the visual detection of the target analyte present in trace or ultratrace amount. Further, detection of the target analyte in aqueous solution, especially in physiological condition, has even more significance owing to the possibility of application potential.

The design of a chemosensor for selective recognition and sensing of an anion is an area of significant importance. [(a) Hu, S.; Guo, Y.; Xua, J.; Shao, S. Org. Biomol. Chem. 2008, 6, 2071, (b) Zeider, F. H. Inorg. Chem. 2008, 47, 1264.]

In this context, sensors that can detect and sense biologically important anions in aqueous environment and under physiological pH are of special significance, owing to their application potential as biological markers. [Khakh, B. S.; North, R.A. Nature, 2006, 442, 527.] . In our earlier communications [(a) Ghosh, A.; Shrivastav, A.; Jose, D. A.; Mishra, S. K.; Mishra, S.; Das, A. Anal. Chem. 2008, 80, 5312. (b) Jose, D. A.; Mishra. S.; Ghosh, A.; Shrivastav, A.; Mishra, S. K.; Das, A. Org. Lett. 2007, 9, 1979.] we have shown that Zn(II)-based coordination complex with pendant

azo-functionality as the reporter group, could be used for staining *Yeast* cells (*Saccharomyces cerevisiae*) through preferential binding to the ATP produced *in situ. Saccharomyces cerevisiae,* a Eukaryotic microbe, derives its energy in the form of ATP and the surface of these Eukaryotic cells is exposed with negatively charged ATP ions. These cells were found to be stained when, exposed to the sensor molecule **1**.**Zn** for approximately five minutes. These preliminary results motivated us to explore the possibility of finding a more efficient receptor molecule as a colorimetric staining agent for Prokaryotic microbes; as well as for studying the growth kinetics. In this context, viable staining agent has more relevance for practical applications.

ATP is a universal energy carrier in biological systems and contributes to biochemical reactions, active transport, nucleic-acid synthesis, muscle activity and the movements of cells. [(a) Ivanova, E. P.; Alexeeva, Y. V.; Pham, D. K.; Wright, J. P.; Nicolau, D. V. Int. Microbiol. 2006, 9, 37. (b) Leevy, W. M.; Johnson, J. R.; Lakshmi, C.; Morris, J.; Marquez, M.; Smith, B. D. Chem. Commun. 2006, 1595. (c) Gordon, J. L. Biochem. J. 1986, 233, 309.] A rise in the cytosolic ATP concentration is a key event in the functioning of membrane depolarization of ATP-dependant $K^+$ channels. Accordingly, measurement of cellular ATP levels in living cells with time is crucial, as this would lead us to assess their metabolic state. In this regard, nature of the staining reagent is important as it should not be toxic to the living system and should be able to sustain the cell proliferation and evaluate its rate. Further, it is well known that *Prokaryotes* and *Eukaryotes* have different cell structure. *Prokaryotes* do not have any membrane-bound organelle, which is present in *Eukaryotes;* e.g *Eukaryotes* have mitochondria. Amongst the *Prokaryotes,* Gram positive (*Gram +ve*) and Gram negative (*Gram -ve*) bacteria have different cell wall structure and chemical composition. [(a) Nelson, D.L.; Cox, M. M.; Lehninger: Principles of Biochemistry, 3rd ed.; Worth Publishers: New York, 2003. (b) Bergey, D. H.; John, G. H.; Noel, R. K.; Peter, H. A. S. Bergey's Manual of Determinative Bacteriology, 9th ed.; Lippincott Williams & Wilkins, 1994.]. Therefore, it would be even more important for the staining agent, if it could effectively delineate the membrane of the smaller bacteria. Thus, to check its wider applicability, we have tested this reagent **(L.Zn)** (Scheme 1, Formula 1A) with two different living cells, *c.a. Bacillus megaterium* and *Pseudomonas fluorescence.* Fresh water is essential for life and methods to monitor bacteria in various sources of fresh water for human consumption, food preparation and industries such as pharmaceutical manufacturing are required for microbiological quality assurance. Among various possibilities, the most popular method is the culture method, where over 24 hours is usually required to obtain results and the bacterial number is sometimes underestimated because some viable bacteria are difficult to culture. Further, quantification and *Yeast* viability are critical for fermentation, [(a) Cahill, G.; Walsh, P.K.; Donnelly, D. J. Am. Soc. Brew. Chem. 1999, 57, 72. (b) Zhang, T.; Fang, H. H. P.; Biotechnol. Lett. 2004, 26, 989.] medical examination, [Biswas, K.; Rieger, K.; Morschhäuser; J. Gene, 2003, 307, 151.], waste water treatment, [Dan, N. P.; Visvanathan, C.; Basu, B. Bioresour. Technol. 2003, 87, 51.] dental biofilm [Millsap, K.W.; M. van der, H. C.; Bos, R.; Busscher, H. J. FEMS, Microbial. Rev. 1998, 21, 321.] and bioengineering systems. [Narvhus, J. A.; Gadaga, T. H. Int. J. Food Microbiol. 2003, 86, 51S.] This necessitates the development of a more rapid and reliable method based on the viable staining of the cells. Though, fluorescence staining reagents are more popular, use of colorimetric staining agents has its own significance owing to the simplicity in detection technique. One can view the stained cells using simple light microscope. In many instances, cells stained with certain fluorescent markers (*e.g.* 4,6-diamidino-2-phenylindole dihydrochloride DAPI) are non-viable. [Baskin, D.S.; Ngo, H.; Didenko, V.V. Toxicol. Sci. 2003, 74, 361.] Moreover, one of the major aspects which, has received relatively less attention is the time profile action of energy dependent process in *Yeast,* which would enable us to monitor the bioprocess in a rapid, easy and accurate manner through ATP binding with the growing microbes i.e. their correlation to growth kinetics response.

More importantly, the zinc complex **(L.Zn)** (Scheme 1, Formula 1A) can be used as a colorimetric staining agent for different biological cells, through binding to the ATP, generated *in situ.* This dye was found to be non-lipophilic in nature and non-toxic to living microbes (*eukaryotes* and *prokaryotes*). Further, stained cells were found to grow in their respective media and this confirmed the maintenance of viability of the microbes even after staining.

Reference may be made to Journal "Shawn C. McCleskey et al. J. Am. Chem. Soc. 2003, 125, 1114.", wherein the use of a combinatorial library of sensors and indicator-displacement assays in the context of a chip-based array allows for the differentiation of structurally similar analytes in aqueous solutions was reported. The coupling of supramolecular chemistry principles with pattern recognition leads to this protocol for sensing. They analysed mixtures of analytes and other phosphate derivatives as a means to explore cross-reactivity between individual library members. Here the drawback of this work is the sensor was selective for triphosphates, but our system is selective for ATP exclusively.

Reference may be made to Journal "Chun Li et al. Angew. Chem. Int. Ed. 2005, 44, 6371", wherein a novel colorimetric and fluorescent sensor was developed for ATP that operates in aqueous solution. This finding will not only extend the application of watersoluble conjugated polymers but also provide a new approach for facile monitoring of ATP. Therefore, they expect that their system will be applicable to routine cellular assays after the rational design of the substituted groups in polythiophenes to improve the membrane permeability of the probe. But here they did not check for live cell staining.

Reference may be made to Journal "Shenliang Wang et al. J. Am. Chem. Soc. 2006, 128, 10380", wherein first turn-on GTP (Guanosine triphosphate) fluorescent sensor was discovered, thus far from a semi-designed diversity directed sensor approach. On the basis of this unprecedented high selectivity of G49 to GTP and its visual green

fluorescence increase, they propose to dub this compound "GTP Green." The main drawback was they did not find any visual colour change which can be detected through naked eye. They only show changes with a fluoro sensor of GTP.

Reference may be made to Journal "Okoh et al. Biochemistry, 2006, 45, 14764", wherein a novel biosensor for inorganic phosphate (Pi) based on the phosphate binding protein of *Escherichia coli* was disclosed. Two cysteine mutations were introduced and labeled with 6-iodoacetamidotetramethylrhodamine. When physically close to each other and correctly oriented, two rhodamine dyes interact to form a noncovalent dimer. In this state, they have little or no fluorescence, unlike the high fluorescence intensity of monomeric rhodamine. The labelling sites were so placed that the distance and orientation between the rhodamines change as a consequence of the conformational change associated with Pi binding. This movement alters the extent of interaction between the dyes. The best mutant of those tested (A17C, A197C) gives rise on average to ~ 18-fold increase in fluorescence intensity as Pi binds. An application of the sensor is demonstrated for measuring ATP hydrolysis in real time as a helicase moves along DNA. Advantages of the new sensor are discussed, both in terms of the use of a rhodamine fluorophore and the potential of this double labeling strategy. Here they only studied with inorganic phosphate; they did not show any change with biological phosphates. This is the main disadvantage of this system.

Reference may be made to Journal "Akio Ojida et al. Chem. Asian J. 2006, 1, 555", described that Nucleoside pyrophosphate (nucleoside PP) derivatives are widespread in living cells and play pivotal roles in various biological events. They report novel fluorescence chemosensors for nucleoside PPs that make use of coordination chemistry. The chemosensors, which contain two Zn(II)-dipicolylamine units, bind strongly to nucleoside PP ($K_a$ > 106 $M^{-1}$ for PP binding) in aqueous solution and sense them by a dual-emission change. Detailed fluorescence and UV/Vis spectral studies revealed that the emission changes of the chemosensors upon binding to nucleoside PPs. This is a unique sensing system based on the anion-induced rearrangement of the coordination. They also demonstrated the utility of these chemosensors in real-time monitoring of two important biological processes involving nucleoside PP conversion: the apyrase-catalyzed hydrolysis of nucleoside PPs and the glycosyl transfer catalyzed by b-1,4-galactosyltransferase. The main problem was that, they did not find any visual colour change which can be detected through naked eye. Everything they show is through luminescence.

Reference may be made to Journal "Akio Ojida et al Angew. Chem. Int. Ed. 2006, 45, 5518", wherein the development of molecular-recognition and sensing systems for biologically important anions which has received considerable attention in recent years was explained. Among various anions, phosphate derivatives, which include nucleoside pyrophosphates such as adenosine triphosphate (ATP), inorganic pyrophosphate, and phosphoproteins, are significant sensing targets because of their pivotal roles in biological systems. ATP is known not only as a universal energy source but also as an extracellular signaling mediator in many biological processes, and protein phosphorylation is recognized as a ubiquitous regulatory mechanism of signal transduction cascades in living cells. Here also they did not show any visual color change. They developed only a new fluorescent chemosensor.

Reference may be made to Journal "Zdenek Kejik et al Chem. Commun., 2006, 1533", wherein one tetrabrucin-porphyrin as a sensor for ATP was reported. They designed and tested the selectivity for ATP in the presence of ADP and AMP. But the shortcoming with this system they found one fluorescence probe for pyrophosphate but there is no explanation that whether it could be used in any biological application.

Reference may be made to Journal "Yasuyuki Tsuboi et al., Applied Surface Science, 2007, 253, 8422", wherein a laser-induced forward transfer (LIFT) of the enzyme luciferase which was explored as a potential technique to be used in the fabrication of a microchip adenosine triphosphate (ATP) sensor was demonstrated. Poly(dimethylsiloxane) (PDMS) was selected as the substrate for deposition of the luciferase. In comparison with other solid substrates, such as glass and polystyrene, it was found that the flexibility of PDMS made it a superior substrate for the immobilization of micro-spots of luciferase. LIFT of luciferase onto a PDMS substrate using a 355 nm laser was successfully carried out, while the bioactivity of the enzyme was maintained. Yellow luminescence ascribed to luciferase was observed from a transferred spot on the PDMS chip from the enzymatic reaction between luciferin and ATP. A microchip ATP sensor was also fabricated by attaching a small photodiode to the PDMS chip. On the basis of the fabricated microchip, the Michaelis-Menten relation between the luminescence intensity from the spot, and the ATP concentration was confirmed. The potential for fabricating biosensors using a combination of the LIFT technique with a PDMS substrate was shown to be very good. Though they did some application in terms of material science but no such application in biology was exhibited by their system.

Reference may be made to Journal "Das et al. Org. Lett., 2007, 9, 1979, and Anal. Chem., 2008, 80, 5312", wherein a novel chromogenic Zn(II) complex with picolyl amine derivative, and its interactions with different biologically important phosphates have been investigated in aqueous solution (pH ~ 7.2). A visual color change can be detected on binding with ATP, whereas no such change is observed when other biologically related anions (AMP, ADP, PPi, or Phosphate) are used. The Zn Complex could be used as a staining agent for different biological cells through binding to the ATP, generated in situ by the mitochondria (in eukaryotes). For prokaryotes (bacteria) the cell membrane takes care of the cells' energy conversion, since they lack mitochondria. ATP is produced in their unique cell structure on the cell membrane, which is not found in any eukaryotes. These stained cells could be viewed with normal light microscopy.

This reagent could even be used for distinguishing the *Gram +ve* and the *Gram -ve* bacteria (prokaryotes).This dye was found to be nonlipophilic in nature and nontoxic to living microbes (eukaryotes and prokaryotes). Further, stained cells were found to grow in their respective media, and this confirmed the maintenance of viability of the microbes even after staining, unlike with many other dyes available commercially. In our earlier work the main problem was solubility, the compound was not soluble in pure water 5% ethanol was needed to solubilise the sensor and at the same time the staining color intensity was less compared to the present invention.

Reference may be made to Journal "Peng Jiang et al. Biochemistry, 2007, 46, 12979", wherein the PII signal transduction proteins are among the most widely distributed signaling proteins in nature, controlling nitrogen assimilation in organisms ranging from bacteria to higher plants was disclosed. PII proteins integrate signals of cellular metabolic status and interact with and regulate receptors that are signal transduction enzymes or key metabolic enzymes. Prior work with *Escherichia coli* PII showed that all signal transduction functions of PII required ATP binding to PII and that ATP binding was synergistic with the binding of R-ketoglutarate to PII. Furthermore, R-ketoglutarate, a cellular signal of nitrogen and carbon status, was observed to strongly regulate PII functions. They demonstrated that in reconstituted signal transduction systems, ADP had a dramatic effect on PII regulation of two *E. coli* PII receptors, ATase, and NRII (NtrB), and on PII uridylylation by the signal transducing UTase/UR. ADP acted antagonistically to R-ketoglutarate, that is, low adenylylate energy charge acted to diminish signaling of nitrogen limitation. By individually studying the interactions that occur in the reconstituted signal transduction systems, they observed that essentially all PII and PII-UMP interactions were influenced by ADP. They also suggest that under certain conditions, the three nucleotide binding sites of the PII trimer may be occupied by combinations of ATP and ADP. In the aggregate, results show that PII proteins, in addition to serving as sensors of R-ketoglutarate, have the capacity to serve as direct sensors of the adenylylate energy charge. They have not studied for the staining of biological cells.

Reference may be made to Journal "Hao Wang et al. Org. Biomol. Chem., 2008, 6, 162", wherein a novel ditopic cholic acid-based fluorescent chemosensor for ATP was described. Its interactions with phosphates, AMP, ADP, ATP, CTP, GTP, and TTP have been investigated. When ATP was added to a 1:1 aqueous $CH_3CN$ solution of the sensor at pH 7.4, a significant decrease in fluorescence was observed, whereas other guest molecules showed a much smaller effect. The complex between the sensor and ATP was confirmed through combined UV, $^1H$, $^{13}C$ and $^{31}P$ NMR spectroscopic methods. The uniqueness of this sensor is that it binds with ATP by 33-124 times more selectively than other nucleotides, as evidenced from the respective binding constants. This sensor is a highly sensitive sensing probe; as little as 30 nM ATP can cause 15% fluorescence quenching of the sensor. Though they got a sensor system binding selectively with ATP, they have not used the sensor for staining biological cells and the sensor did not work in physiological conditions.

Reference may be made to Journal "Tae-Hyuk Kwon et. al. Chem. Eur. J. 2008, 14, 9613", wherein an ensemble sensor system that exhibited selective luminescence enhancement upon binding to thymidine 5'-triphosphate (TTP) in HEPES buffer over other nucleotides was reported. The ensemble system consisted of an energy acceptor and an energy donor. Among the nucleotides, the selective recognition and luminescence enhancement for TTP was achieved by the strong binding of the thymine unit to $Zn^{2+}$-cyclen (cyclen = 1,4,7,10-tetraazacyclododecane) and intermolecular energy transfer between the mCPand FIrpic moieties. They did not show any biological application with this sensor.

Reference may be made to Journal "Akihiko Ishida et al. Anal Bioanal Chem, 2008, 392, 987", wherein the development of a colorimetric method for the assay of ATP, using enzymatic cycling and Fe(III)-xylenol orange (XO) complex formation was described. The colour characteristics of the Fe(III)-XO complexes, which show a distinct color change from yellow to purple, assist the visual observation in screening work. In this method, a trace amount of ATP was converted to pyruvate, which was further amplified exponentially with coupled enzymatic reactions. Eventually, pyruvate was converted to the Fe(III)-XO complexes through pyruvate oxidase reaction and Fe(II) oxidation. As the assay result, yellow or purple colour was observed: a yellow color indicates that the ATP concentration is lower than the criterion of the test, and a purple color indicates that the ATP concentration is higher than the criterion. The method was applied to the assay of ATP extracted from *Escherichia coli* cells added to cow milk. They have neither studied for the staining of biological cells nor had they done any cell growth dynamics.

Reference may be made to Journal "Yimin Sun et al. Org. Biomol. Chem. 2008, 6, 3044", wherein a new 4-amino-1,8-naphthalimide-based fluorescent chemosensor bearing a guanidiniocarbonyl pyrrole moiety was disclosed. The sensor displays a selective fluorescent enhancement with pyrophosphate over ATP, ADP, AMP and other inorganic anions in aqueous solution. The main drawback with the system is that they have not checked their system with any biological cells.

Reference may be made to Journal "Itaru Hamachi et al J. Am. Chem. Soc. 2008, 130, 12095" wherein fluorescence sensing with small molecular chemosensors is a versatile technique for elucidation of function of various biological substances was described. This fluorescent chemosensor developed for nucleoside polyphosphates such as ATP using metal-anion coordination chemistry. The chemosensor comprised of the two sites of 2,2'-dipicolylamine (Dpa)-Zn(II) as the binding motifs and xanthene as a fluorescent sensing unit for nucleoside polyphosphates. The chemosensor 1-2Zn(II) selectively senses nucleoside polyphosphates with a large fluorescence enhancement ($F/Fo$ >

15) and strong binding affinity, whereas no detectable fluorescence change was induced by monophosphate species and various other anions. The 'turn-on,' fluorescence of 1-2Zn(II) is based on a new mechanism, which involves the binding-induced recovery of the conjugated form of the xanthene ring from its nonfluorescent deconjugated state which was formed by an unprecedented nucleophilic attack of zinc-bound water. The selective and highly sensitive ability of the sensor to detect nucleoside polyphosphates enables its bioanalytical applications in fluorescence visualization of ATP particulate stores in living cells, demonstrating the potential utility of the sensor. They studied some biological cell staining, they showed cell staining through confocal microscope, but with our present invention we can see the stained cells through simple light microscope.

Reference may be made to Journal "Hongmei Wu et al. Inorg. Chem. 2009, 48, 408", wherein a metallohelical triangles consisting of chromophore units and hydrogenbonding trigger sites which were prepared by modulating two tridentate $N_2O$ units containing amide groups within a central benzene ring at the meta sites, for the selective detection of adenosine trisphosphate in aqueous media over other ribonucleotides was developed. Though they got a sensor system binding selectively with ATP, they have not studied the sensor as a staining agent with biological cells.

Reference may be made to Journal "Aiko Nonaka et al. Org. Biomol. Chem. 2008, 6, 3621", wherein a new strategy for the fluorescence detection of multi-phosphates in aqueous solution. ZnII-DPA(DPA = dipicolylamine)-appended phenylboronic acid forms an assembly with alizarin dye, in which the dye binds favourably to the coordinated zinc(II) in the ZnII-DPA moiety was disclosed. Addition of pyrophosphate (PPi) as a putative analyte causes reorganization of the complex to produce an alternative boronate ester assembly, which causes an increase in fluorescence, detectable by the naked eye. It is interesting to note that the system exhibited PPi-selectivity over other phosphates. The deficiency of this system is that, they found a sensor which binds with different phosphates which is not selectively binding with a phosphate hence it is not a selective sensor for ATP.

**Comparison of the Zn (II) based colorimetric sensor L.Zn (Scheme 1, Formula 1A) of present invention with Zn (II) based colorimetric sensor 1.Zn (Scheme 1b) described in Anal. Chem. 2008, 80, 5312-5319 and Org. Letts. 2007, 9,1979-1982:**

Zn (II)-based complex [1.Zn (Scheme 1b)] as described in earlier communications *(*Organic Letters, 2007, Vol. 9, No.10, 1979-1982 and Anal. Chem. 2008, 80, 5312-5319) of the inventors is different from [L.Zn (Scheme 1, Formula 1A)] described in the present invention. Observation reported in these two articles showed that 1.Zn had crucial limitations in terms of its usability in pure aqueous solution; as any staining agent that may find practical application has to be completely soluble in aqueous solution. 1.Zn was insoluble in water and one has to use certain amount of alcohol for solubilizing this and then this alcoholic solution of this reagent could be used for staining purpose. Though, the complex was found to be non toxic to the prokaryotic and eukaryotic cells, use of alcohol was not desirable or allowed for practical cell staining experiments, as it may have some adverse effect on living organism. The present invention has been able to overcome this problem.

In this patent application we have reported another Zn(II)-based complex **L.Zn** of formula 1A which could bind ATP with associated changes in color that could be detected with naked eyes.

This new reagent is completely soluble in water and thus, is suitable and most appropriate for any practical application in regard to cell staining experiments. Further, on binding to ATP, the absorption maxima for this complex shifts by 40 nm (from 463 nm to 503 nm) and this caused a more distinct and sharper color changes as compared to the reagent that we have reported in our earlier publications (1.**Zn**).

For our earlier reagent (1.**Zn**) shift was only 21 nm in ethanol-aqueous buffer medium. This larger shift of 40 nm for the present Zn (II)-complex **L.Zn** (reported in this patent application) is crucial for spectral measurements and thus, crucial for cell growth dynamics studies.

Further, for an appropriate sensor it is desirable to have an optimum binding constant value. Binding constant for the present Zn (II)-complex, reported in this patent application, is lower (978 $\pm$ 4) $M^{-1}$ than that we have reported earlier (1130 $\pm$ 6) $M^{-1}$) for **(1.Zn).** This will make this sensor to bind ATP more reversibly as compared to the previously reported Zn(II)-complex **(1.Zn).**

More importantly, higher solubility of this Zn(II)-reagent could be achieved through use of $\alpha$-cyclodextrin ($\alpha$-CD)(a = **L.Zn** only, b = **L.Zn** + $\alpha$-CD in Figure 8). In presence of $\alpha$-CD the solubility of L.Zn in aqueous solution increases, as we see in Figure 8, with addition of $\alpha$-CD the color intensity increases which indicate that more amount of **L.Zn** goes in to the solution. No such observation was reported for the earlier reagent **1.Zn.** Use of $\alpha$-CD is common as drug carrier and is known to be completely non-toxic to the living cell. Thus, use of $\alpha$-CD, in combination with Zn(II)-complex (reported in this patent application) provides the opportunity to use this sensor molecule in higher concentration, which could be helpful for achieving better color change (Figure 13, D1 without $\alpha$-CD and D2 with $\alpha$-CD) and faster staining of the living cells.

Using the present Zn(II)-reagent **(L.Zn)** in presence of $\alpha$-CD, eukaryotic and prokaryotic (*Gram +ve* / *Gram -ve*) cells could be stained with only 2 minutes incubation; while for the previous reagent (1.**Zn**) cells were needed to be

incubated for 10 minutes for color development. Further, intensity of color of the stained cell (using present Zn(II)-complex was much more intense as compared to that reported earlier. This could only be achieved due the structural variation (use of the azamacrocyclic ligand in the present study instead of bispicolyl amine as reported in **1**.**Zn**) (Scheme 1b).

Present Zn(II)-complex is found to be more selective towards ATP as compared to the CTP and the associated colour change with CTP is almost negligible (Figure 2a). **Synthetic variation:** According to the synthetic procedure, ligand that was used for the synthesis of the previously reported Zn(II)-complex (**1**.**Zn**), was synthesized by reacting dipicolyl amine and 4-(4-dimethylamino-phenyl azo) benzene sulphonyl chlorides (1:1.05 mole equivalent) at 0°C to achieve the optimum yield. For synthesis of the present ligand **(L)** 1,4,8,11-tetra azacyclotetradecane was allowed to react with 4-(4'-dimethylamino-phenyl azo) benzene sulphonyl chloride (1:1 mole equivalent) at room temperature (30°C) for 10 hours and then reflux for 1 hr. Any attempt to increase the concentration of 4-(4-dimethylamino-phenyl azo) benzene sulphonyl chlorides will lead to a multiple product and impurity and it was very difficult to get read off these impurities. Further, reaction temperature is also crucial for achieving a reasonable yield. Reaction at 0°C would lead to a very poor yield for the desired product in the present study. Further, in the present study, purification for the Zn(II)-complex was achieved (removal of the unreacted organic reagent) through washing with $CHCl_3$, which was not possible for the previous study due to its higher solubility in this organic solvent. This purification step made purification of the desired Zn(II)-complex **(L.Zn)** much easy and helped us to isolate it in the purest form. Further, Zn(II)-center in the previous complex (1.**Zn**) is coordinated to three nitrogen atom of the bispicolyl amine functionality and this restricts the possibility of the binding of three phosphate oxygen to the Zn(II)-center. While in the present system **(L.Zn),** four N-atoms of the tetrazamacrocyclic moiety is bound to the Zn(II)-center with Zn(II)-ion remains above the plane of the coordinating N-atoms and this helps coordination of three phosphate O-atoms to the Zn(II)-center (Scheme 1a), which eventually leads to the better spectral and visible color change on binding to ATP.

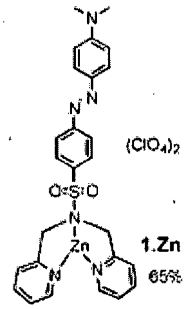

**1.Zn**
65%

Application Potential of the present Sensor Molecule **(L.Zn)** and limitation of the previously reported molecule (1.**Zn**)**:** This type of colorimetric sensor molecules could be very useful for research on Cystic fibrosis as this allows the quantitative detection of ATP produced *in-situ,* which reveals the energy efficiency of the cells. This measurement has a critical significance as ATP regulates most of the crucial proteins which are important for Cystic fibrosis. Further, these type of colorimetric sensors could also be used with suspension cells for experiments with flow cytometry as a way to quantitate amount of ATP. Obviously the binder has to be selective towards ATP and active in aqueous solutions.

For such applications it is essential to avoid use of any alcohol or other organic solvents. Major limitation of the previously reported (D1 and D2) compound **1**.**Zn** is that one has to use certain amount of alcohol and that limits the possibility of using this reagent for practical purpose. While one uses pure aqueous solution the recent complex **(L.Zn)** for cell staining studies. One of the major components is uncommon for synthesizing this **(L.Zn)** complex (when compared to the previous complex **1**.**Zn**), which adds to the difference or more precisely the improved solubility and staining property. In the synthetic procedure, there is an apparently minute, but crucial difference, which is crucial in achieving the desired compound **L.Zn**.in optimum yield. Few crucial adverse effects of alcohol: One effect of drinking alcohol is "blood-sludging" where the red blood cells clump together causing the small blood vessels to plug up, starve the tissues of oxygen, and cause cell death. This cell death is most serious, and often unrecognized, in the brain. Alcohol is not digested like other foods, instead of being converted and transported to cells and tissues; it avoids the normal digestive process and goes directly to the blood stream. About 20 percent of the alcohol is absorbed directly into the blood through the stomach walls and 80 percent is absorbed into the bloodstream through the small intestine.

## OBJECTIVE OF THE INVENTION

The main objective of the present invention is to prepare a Zn(II) based colorimetric sensor (Scheme 1, Formula 1A) and develop a process for its use as a viable staining agent for living cells.

Another object of the present invention is to develop a methodology for sensing using a chemosensor that binds selectively to adenosine triphosphate (ATP) with change in color that allows its visual detection.

Still another objective of the present invention is to standardize a method for detection of ATP in aqueous solution or physiological conditions.

Yet another objective of the present invention is to find a suitable methodology for improving solubility of the chemosensor in water.

Yet another objective of the present invention is to develop suitable methodology for detection of ATP in living organism (microbes) like *Yeast* (Eukaryotic) cell and *Gram +ve* and *Gram -ve* bacterial (Prokaryotic) cells using optical light microscope.

Yet another objective of the present invention is to explore the possibility of using this chemosensor for distinguishing *Gram +ve* and *Gram -ve* bacteria.

Yet another objective of the present invention is to test the viability of the chemosensor.

Yet another objective of the present invention is to establish a methodology for studying the cell growth dynamics.

## SUMMARY OF THE INVENTION

Accordingly, present invention provides a compound of formula 1A.

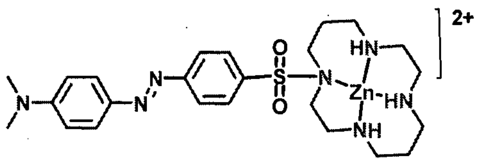

**Formula 1A**

In an embodiment of the present invention, said compound is non-lipophilic in nature. In another embodiment of the present invention, said compound is colorimetric sensor useful as a viable staining agent for living cells.

In yet another embodiment of the present invention, said living cells are selected from Prokaryotic and Eukaryotic cells.

In yet another embodiment of the present invention, prokaryotic cells used are *Bacillus* species and *Pseudomonas* species.

In yet another embodiment of the present invention, Eukaryotic cells used is yeast *(Saccharomyces cerevisiae).*

In yet another embodiment of the present invention, process for the preparation of the compound of formula **1A** comprising the steps of:

i. dissolving 1,4,8,11-tetraazacyclotetradecane in a solvent under mild stirring at the rate of 300 to 600 rpm (rotation per minute) at temperature in the range of 25-30°C;
ii. keeping the solution as obtained in step (i) in ice at temperature in the range of 2 to 5°C;
iii. adding triethyl amine base in the solution as obtained in step (ii) at the rate of 0.5 to 1 mL/minute under mild stirring at the rate of 300 to 600 rpm;
iv. adding separately prepared solution of 4-(4-dimethylamino-phenyl azo) benzene sulphonyl chloride in tetrahydrofuran in the solution as obtained in step (iii) at a rate in the range of 15 to 60 mL/hr at a temperature in the range of 0 to 5°C;
v. stirring of the resulting mixture as obtained in step (iv) in the temperature range of 25 to 30°C for a period in the range of 5 to 10 hours;
vi. refluxing the mixture as obtained in step (v) for a period in the range of 20 to 60 minutes at temperature in the range of 65 to 70°C;
vii. filtering the mixture as obtained in step (vi) to separate the precipitate of ligand;
viii. washing and drying the precipitate of ligand as obtained in step (vii);
ix. dissolving the ligand as obtained in step (viii) in alcohol at temperature in range of 20 to 25°C;
x. adding zinc nitrate solution (0.3 to 0.8 mole equivalent of the ligand) in the solution of ligand as obtained in step (ix) at a temperature in the range of 20 to 25°C;
xi. stirring the solution as obtained in step (x) at temperature in the range of 20 to 25°C for a period in the range of 12 to 24 hours;
xii. agitating the solution as obtained in step (xi) at a temperature in the range of 10 to 20°C for a period in the range of 2 to 5 hours;
xiii. filtering and washing the precipitate as obtained in step (xii) with chloroform to obtain compound of formula 1A.

In yet another embodiment of the present invention, said solvent are selected from the group consisting of

chloroform, methanol, dichloromethane and tetrahydrofuran (THF). In yet another embodiment of the present invention, alcohol used is selected from methanol and ethanol.

In yet another embodiment of the present invention, process for staining living cells using the compound of formula 1 and the said steps comprising of:

a. cultivating the (i) *Saccharomyces* species (MTCC 5548) in glucose yeast extract agar (GYE) media, (ii) *Bacillus* species (MTCC 5549) in Nutrient broth and (iii) *Pseudomonas* species (MTCC 5550) in King's B medium at a temperature in the range of 30 to 40°C for a period in the range of 6 to 36 hours;
b. incubating the cells of step (a) with $0.6x10^{-4}$ to $1.2x10^{-4}$ M compound of formula 1A in the temperature range of 28 to 38°C for a period in the range of 5 to 20 minutes;
c. fixing the incubated sample as obtained in step (b) with gluteraldehyde in buffer at pH in the range of 7.4 to 7.6 for period in the range of 10 to 12 hours at a temperature in the range of 28 to 38°C;
d. washing the sample as obtained in step (c) with said buffer for period in the range of 0.5 to 1.0 hour;
e. post fixing the sample as obtained in step (d) with osmium tetraoxide and buffer and washing with the said buffer;
f. removing water from sample as obtained in step (e) by graded water ethanol series;
g. rinsing the sample as obtained in step (f) in said buffer and coating with gold in a sputter coater to obtain stained specimen for viewing under the simple microscope;
h. distinguishing the living cells with different intensity of color and shape of the stained cells.

In yet another embodiment of the present invention, buffer used is phosphate buffer.

In yet another embodiment of the present invention, binding of sensor and living cell is confirmed by Scanning Electron Microscopy (SEM) analysis.

In yet another embodiment of the present invention, viability of the cells after staining with the dye is proved by observing binary fission in tested microbes by hanging drop method in concavity slide revealing the occurrence of cell division.

In yet another embodiment of the present invention, reversible binding of compound of formula 1A is checked by destaining the cells with sodium citrate 20% (w/v) solution through light microscope and UV-Vis spectroscopy.

In yet another embodiment of the present invention, staining of the living cell by compound of formula 1A is carried out due to the formation of hepta coordinated species of sensor with ATP present on the cell wall of the living cells where the three phosphate groups are coordinated with Zn and obtained $^{31}$PNMR shift for all the three phosphorus atoms.

In yet another embodiment of the present invention, absorption spectral titration curve of compound of formula 1A shows 40 nm spectral shift after addition of ATP and binding constant of compound with ATP is (978 $\pm$ 4) $M^{-1}$.

In yet another embodiment of the present invention, solubility of compound of formula **1A** in water is increased about 7 to 7.5 times in the presence of $\alpha$-CD (cyclodextrin).

In yet another embodiment of the present invention, intensity of the staining of compound of formula **1A** is increased in the presence of $\alpha$-CD (cyclodextrin) by showing absorption maxima for Zn(II) based colorimetric sensor **L.Zn** tend to shift by 5 nm on addition of aqueous solution of $\alpha$-CD (cyclodextrin).

In yet another embodiment of the present invention, staining with compound of formula 1A take place in 10-20 minutes and in the presence of $\alpha$-CD (cyclodextrin) it take place in 1 second to 2 minute showing speed of the staining of compound of formula 1A is increased in the presence of $\alpha$-CD (cyclodextrin).

In yet another embodiment of the present invention, SEM images of *Saccharomyces species cell* surfaces without dye were found to be smooth in contrast to with dye were found to be rough.

In yet another embodiment of the present invention, SEM images of *Gram +ve* bacteria without dye were found to be smooth and with dye were rough.

In yet another embodiment of the present invention, SEM images of *Gram -ve* bacteria without dye were found to be smooth and with dye were rough.

In yet another embodiment of the present invention, stained cells for *Gram +ve* bacteria appeared longer as compared to those of the stained *Gram -ve* bacteria.

In still another embodiment of the present invention, 1,4,8,11-tetraazacyclotetradecane is in the concentration range of 6 to 15 milli molar.

In yet another embodiment of the present invention, the rate of addition of triethylamine is selected from the range of 0.5 to 1.0 mL/min.

In still another embodiment of the present invention, 4-(4'-dimethyl amino phenyl azo)-benzene sulphonyl chloride is in the concentration range of 15 to 20 mM.

In still another embodiment of the present invention, rate of addition of 4-(4-dimethyl amino phenyl azo)-benzene sulphonyl chloride is selected from the range of 15 to 60 mL/hour.

In still another embodiment of the present invention, the reaction mixture is stirred for a period in the range of

5 to 10 hours while maintaining the temperature in the range of 25 to 30°C.

In still another embodiment of the present invention, the reaction mixture is refluxed for a period in the range of 20 to 60 minutes.

In still another embodiment of the present invention, the ligand so prepared is dissolved in solvent while keeping the concentration in the range of 14 to 20 mM and temperature in the range of 20 to 30°C.

In still another embodiment of the present invention, the zinc nitrate solution is used in the range of 0.3 to 0.8 mole equivalent of the ligand.

In still another embodiment of the present invention the reaction mixture of the ligand and the zinc nitrate solution is stirred for the period in the range of 12 to 24 hours at a temperature range of 20 to 30°C.

In still another embodiment of the present invention the final reaction mixture is kept in static position at a temperature in the range of 10 to 20°C for a period in the range of 2 to 5 hours.

In still another embodiment of the present invention, cultivated species were incubated with the sensor in a concentration range of 0.1-0.15 mM at a temperature in the range of 28 to 38°C and for a period in the range of 5 to 20 minutes.

In still another embodiment of the present invention, biological samples were stained with sensor.

In still, another embodiment of the present invention, *Yeast* cells (Eukaryotic) *Gram* +ve and *Gram -ve* bacteria (Prokaryotic) could be distinguished based on the intensity of the color of the stained cells when stained with the same sensor.

In still, another embodiment of the present invention, colonies of the test microbes after exposure were grown by streaking on nutrient agar plate to check their viability.

## BRIEF DESCRIPTION OF THE DRAWINGS

**Scheme 1** represents the synthesis of Zn (II)-azamacrocyclic complex (**L.Zn**) (Scheme 1, Formula 1A) and also represents the binding mode of **L.Zn** with ATP (Scheme 1, Formula 1B).

**Scheme 2** represents the inclusion mechanism of **L.Zn** (Scheme 1, Formula 1A) with $\alpha$-CD ($\alpha$- cyclodextrin).

**Figure 1** represents absorbance spectra of **L.Zn** (Scheme 1, Formula 1A) (8.87 $\mu$M) in HEPES (N-(2-hydroxyethyl)-piperazine-N'-2-ethanesulfonic acid) buffer solution (pH ~ 7.2) at 25°C in presence of various anions (~17.0 mM); 1: ATP; 2: CTP; 3: ADP; 4: $F^-$, $Cl^-$, $Br^-$, $I^-$, $CH_3COO^-$, $H_2PO4^-$, PPi, $SO_4^{2-}$, $NO_3^-$, $CN^-$, AMP, $SCN^-$.

**Figure 2** represents change in color of **L.Zn** (Scheme 1, Formula 1A) (8.87 $\mu$M) in aqueous solution in absence and presence of various phosphate ions. From left to right: Blank, with added $H_2PO_4^-$, PPi, AMP, ADP, ATP, CTP (Anion concentration ~500 $\mu$M).

**Figure 3** represents a graph showing absorption spectral titration curves when ([ATP] = 0 - 17.28 mM) is added to the aqueous solution of **L.Zn**(II)-compound (Scheme 1, Formula 1A) (9.46 $\mu$M) at pH 7.2 (10 mM HEPES buffer solution) at 25°C.

**Figure 4** represents a graph showing absorption spectral titration curves when ([CTP] = 0 - 10.17 mM) is added to the **L.Zn** (II)-compound (Scheme 1, Formula 1A) (1.79 $\mu$M) at pH 7.2 (10 mM HEPES buffer solution) at 25°C.

**Figure 5** represents a graph showing absorption spectral titration curves when ADP ([ADP] = 0 - 6.55 mM) is added to the **L.Zn** (II)-compound (Scheme 1, Formula 1A) (8.86 $\mu$M) at pH 7.2 (10 mM HEPES buffer solution) at 25°C.

**Figure 6A** represents a graph showing $^{31}$P NMR spectra recorded in $D_2O$ using 500 MHz Bruker (Model noAdvance-DPX-500) Instrument to reveal the mode of binding of **L.Zn-ATP** (Scheme 1, Formula 1B); only ATP (A) and ATP with Zn(II)-complex (B), **L.Zn** (Scheme 1, Formula 1A) at 25°C.

**Figure 6B** represents a graph showing $^{31}$P NMR spectra recorded in $D_2O$ using 500 MHz Bruker (Model noAdvance-DPX-500) Instrument to reveal the mode of binding of **L.Zn-ATP** (Scheme 1, Formula 1B); only CTP (C) and CTP binding with Zn(II)-complex (D), **L.Zn** (Scheme 1, Formula 1A) at 25°C.

**Figure 7** represents a graph showing the results of ESI (electrospray ionization) mass analysis when the compound (7.2 $\mu$M) and excess ATP (900 $\mu$M) are present conforming the formation of **L.Zn-ATP** (Scheme 1, Formula 1B). Molecular mass for **L.Zn-ATP** (Scheme 1, Formula 1B) (monosodium salt) is 1081. (Instrument used: Thermo Finnigan LCQ™ Advantage MAX quadrupole ion trap instrument, San Jose, Calif., USA)

**Figure 8** represents the systematic spectral change of **L.Zn** (Scheme 1, Formula 1A) (8.3 $\mu$M) with varying [$\alpha$-CD] (0-2.3mM) in aqueous solution at 25°C. Insert is the photograph of the colour of the saturated solution of **L.Zn** (Scheme 1, Formula 1A) in (a) absence and (b) presence of $\alpha$-CD in double distilled and deionised water.

**Figure 9A** represents a Light Microscopy images of (A) unstained *Yeast* cells and (B) *Yeast* cells stained with **L.Zn** (Scheme 1, Formula 1A) ($1.16 \times 10^{-4}$ M) and (C) **L.Zn** (Scheme 1, Formula 1A) ($5 \times 10^{-4}$ M) and $\alpha$ -CD ($2.92 \times 10^{-3}$ M) at 25°C.

**Figure 9B** represents a Light Microscopy images of *Gram +ve* bacteria (A) unstained (B) stained with **L.Zn** (Scheme

1, Formula 1A) ($0.66x10^{-4}$ M) and (C) **L.Zn** (Scheme 1, Formula 1A) ($0.5x10^{-3}$ M) and α-CD ($2.92x10^{-3}$ M) at 25°C.

**Figure 9C** represents a Light Microscopy images of *Gram -ve* bacteria; (A) unstained, (B) stained with **L.Zn** (Scheme 1, Formula 1A) ($0.66x10^{-4}$ M) and (C) **L.Zn** (Scheme 1, Formula 1A) ($0.5x10^{-3}$ M) and α-CD ($2.92x10^{-3}$M) at 25°C.

**Figure 10A** represents a absorption spectra of (a) **L.Zn** (Scheme 1, Formula 1A) (2.97 μM)/ **L.Zn** (Scheme 1, Formula 1A) in presence of ATP (13.6 mM)/ ATP (13.6 mM) and sodium citrate solution (20% (w/v)), (b) **L.Zn** (Scheme 1, Formula 1A) (7.1 μM)/ in presence of *Yeast*/ **L.Zn** (Scheme 1, Formula 1A) (7.1 μM) + in presence of *Yeast* cells and to this sodium citrate solution (35% (w/v)) was added. Spectra were recorded at 25°C.

**Figure 10B** represents a Light Microscopy images of *Yeast* (A) stained with **L.Zn-**α.CD (0.5 mM) [α.CD ] = (2.92 mM) (B) stained with **L.Zn** (66 μM) and (C) **L.Zn-**α.CD stained *Yeast* cell after treatment with sodium citrate (20%W/V), (D) **L.Zn** stained *Yeast* cell after treatment with sodium citrate (20%W/V).

**Figure 11** represents SEM images of (A) blank *Yeast* cells, (B) *Yeast* cells treated with **L.Zn** complex (Scheme 1, Formula 1A), (C) blank *Gram -ve* bacterial cells, (D) *Gram* -ve bacterial cells treated with **L.Zn.** (Scheme 1, Formula 1A) complex, (E) *Gram + ve* bacterial cells and (F) *Gram +ve* bacterial cells treated with **L.Zn** (Scheme 1, Formula 1A) complex. [**L.Zn**]=0.116 mM.

**Figure 12** represents growth curve of (a) *Saccharomyces cerevisiae,* (c) *Pseudomonas fluorescence* and (e) *Bacillus megaterium* obtained from change in cell count with time. Relative change in absorbance at 600 nm for aqueous culture medium of (b) *Saccharomyces cerevisiae,* (d) *Pseudomonas fluorescence* and (f) *Bacillus megaterium* in presence of **L.Zn** (Scheme 1, Formula 1A) (D-1) (0.106 mM) and **L.Zn** (Scheme 1, Formula 1A) (0.164 mM) and α-CD (0.938 mM) (D-2). Studies were performed at 25°C.

**Figure 13** represents the Light microscopy images of a *Yeast* cell stained with **L.Zn** (Scheme 1, Formula 1A) [(D-1) (0.106 mM) and **L.Zn** (Scheme 1, Formula 1A) (0.164 mM) and α-CD (0.938 mM) (D-2)] and monitored at different time interval.

## DETAIL DESCRIPTION OF THE INVENTION

Present invention provides that the intermediate ligand (E)-4-((4-(1,4,8,11-tetraazacyclotetradecan-1-ylsulfonyl)phenyl)diazenyl)-N,N-dimethylaniline (L) (Scheme 1, Formula 2) was synthesized by reaction of 1,4,8,11-tetraazacyclotetradecane with (E)-4-((4-(dimethylamino)phenyl)diazenyl) benzene-1-sulfonyl chloride. This was further purified before using it for reaction with $Zn(NO_3)_2$ in non aqueous solvent for synthesis of the corresponding Zn(II)-complex **(L.Zn(II))** (Scheme 1, Formula 1A). This complex was found to bind triphoshates like ATP and CTP either in neutral aqueous solution or in solution with physiological pH with associated change in color that could be detected in naked eyes. This opens up the possibility of using this Zn(II)-complex as a possible staining agent for living Eukaryotic and Prokaryotic cells. This Zn(II)-reagent can also be used for detection of CTP, but affinity of this complex towards CTP is much lower as compared to the ATP. Based on these findings, the present invention was gradually developed through various analytical techniques involving different (chemical and biological) assay methods. Present invention provides a Zn(II)-azamacrocycle-based complex, L.Zn, (Scheme 1, Formula 1A) having selectivity for ATP.

The present invention provides a method for the preparation of Zn(II) based colorimetric sensors which comprises of steps:

i. dissolving 1,4,8,11-tetraazacyclotetradecane in the concentration range of 6 to 15 mM. with solvents in the volume range of 50 to 100 mL and the solvent used in the range of chloroform, methanol, dichloromethane, tetrahydrofuran and the like;

ii. keeping the solution of step (i) in the temperature range of 2 to 5°C, triethyl amine base was added at a rate of 0.5 to 1 mL/minute under mild stirring and then 15 to 25 mM solution of 4-(4-dimethylamino-phenyl azo) benzene sulphonyl chlorides was added at a rate in the range of 15 to 60 mL/hour;

iii. stirring of the resulting mixture of step (ii) in the temp range of 25 to 30°C for a period in the range of 5 to 10 hours;

iv. refluxing the mixture for a period in the range of 20 to 60 minutes;

v. filtering and then washing with THF of the precipitate and then drying the solid to obtain the ligand;

vi. dissolving the ligand in alcohol to have a concentration in the range of 14 to 20 mM at temperature in range of 20 to 25°C;

vii. adding zinc nitrate solution in water in the range of 0.3 to 0.8 mole equivalent of the ligand to the solution prepared in step (vi) at temp in the range of 20 to 25°C;

viii. stirring the solution of step (vii) at the same temperature range for a period in the range of 12 to 24 hours;

ix. agitating the resulting solution of step (viii) at the at the temp range of 10 to 20°C for a period of 2 to 5 hours;

x. filtering, washing the precipitate with chloroform to obtain the sensor;

xi. cultivating the isolated (i) *Saccharamyces* species in GYE media, (ii) *Bacillus* species in Nutrient broth and (iii) *Pseudomonas* species in King's B medium at a temperature range of 30 to 40°C for a period in the range of 6 to 36 hours;

xii. incubating 100 μl of each cultivated species obtained in step (xi) with 100 μl of sensor obtained in step (x) having a concentration in the range of 0.1 - 0.15 mM and in the temperature range of 28 to 38°C for a period in the range of 5 to 20 minutes;
xiii. staining the sample with sensor for viewing under the simple microscope;
xiv. distinguishing the *Gram +ve* and *Gram -ve* bacteria with different intensity of color;
xv. growing the colonies of test microbes from steps (xi) to (xiv) when streaked on the nutrient agar plate.

**Synthesis of compound L (Scheme 1, Formula 2)**

1,4,8,11-tetraazacyclotetradecane (cyclam) (247 mg, 1.23 mmole) was dissolved in dry tetra hydrofuran (40mL) in a 250 mL Round Bottom RB Flask. A solution of 4-(4-Dimethylamino-phenylazo)-benzenesulphonyl chloride (400 mg, 1.23 mmole) and triethyl amine ($Et_3N$) (1mL) in tetrahydrofuran (10-15 mL) was added to the above solution in ice cold condition. Then, the resulting mixture was allowed to stir 25°C to 30°C for 10 hour and then it was further refluxed for 1 hour. The orange precipitate thus, obtained was filtered and washed with tetrahydrofuran. Finally the solid residue (**L**) was dried; Yield: 75 %.

**Synthesis of compound L.Zn (Scheme 1, Formula 1A)**

A solution of L (200 mg; 0.41 mmole) was dissolved in 25 mL methanol in a 100 mL one neck flask. To this 1.0 mL of zinc nitrate solution in water (equivalent to 148.69 mg of $ZnNO_3.6H_2O$, 1.2 mole equivalent) was added. The resultant solution was stirred at 25°C to 30°C for 24 hour. The reaction mixture was kept in refrigerator 4°C for 5 hour and a violet colored precipitate appeared. This was collected through filtration using Gooch (no. 3) crucible and was washed several times with $CHCl_3$; Yield: 65%.

**Biological sample preparation**

The microbes that have been used in staining studies are *Pseudomonas sp.* (MTCC 5550) isolated from CSMCRI garden soil, (21.7587325N 72.1443897E) *Bacillus sp.* (MTCC 5549) isolated from Arabian Sea (Verawal coast, 20.95973N 70.27932E), *Saccharomyces sp.* (MTCC 5548) isolated from curd.

The isolates of *Saccharomyces sp.* were cultured in GYE (glucose yeast extract agar) media, *Bacillus sp.* in Nutrient broth, *and Pseudomonas sp.* in King's B medium for 24 hour at 37°C. 100 μl of this was taken and was allowed to incubate at 30 to 40°C along with 100 μl of **L.Zn** (Scheme 1, Formula 1A) ($1.16x10^{-4}$ M) solution. The samples were fixed overnight at room temperature 25-30°C with 2 % (v/v) glutaraldehyde in 0.1 M phosphate buffer at pH 7.5. The samples were then washed with 0.1 M phosphate buffer (pH 7.5) at room temperature 25-30°C for 1 hour. Post-fixation was carried out in 2 % (w/v) osmium tetroxide ($OsO_4$) in the same buffer and was washed once with 0.1 M phosphate buffer for 20 minutes. Then, the water was removed by a graded water-ethanol series; 25 % ethanol for 15 min, 50 % ethanol for 15min, 75 % ethanol for 30 min, 90 % ethanol for 60 min, absolute alcohol for 30 min. The specimens were rinsed in buffer and coated with gold in a sputter coater (Polaron SC7620) prior to microscopy. The material was examined in a Scanning Electron Microscope (SEM) LEO 1430 VP at an accelerating Voltage of 15 kV.

SEM images of blank and stained eukaryote (*Yeast*), *Gram +ve* and *Gram -ve* cells have been recorded (Figure 11) to reveal the change(s) in the morphology of the outer surface of the cells on staining, as maximum concentration of ATP is released through mitochondria in the periplasmic space and then gets excreted to the cell surface. Several ATP binding cassette proteins (eg Periplasmic Protein (PEP), Mammalian AMP-activated protein kinase (AMPK)) are also reported to accumulate in the plasma membrane. [(a) Nelson, D. L.; Cox, M. M.; Lehninger: Principles of Biochemistry, 2003 3rd Edition, WorthPublishers. NY; Bergey, (b) John, D. H.; Noel, G. H.; Peter, R. K.; Bergey's, H. A. S. Manual of Determinative Bacteriology, 1994, 9th ed., Lippincott Williams & Wilkins]. SEM images of *Yeast* cells without dye were found to be smooth in contrast to the images of the *Yeast* cells with dye, where stained cell surfaces were found to be rough. This revealed the presence of the extraneous material i.e **L.Zn** (Scheme 1, Formula 1A) adhering to the cell. Changes on the cell surfaces on binding of the dye **L.Zn** (Scheme 1, Formula 1A) were also evident from the SEM images recorded for *Gram -ve* and *Gram +ve* bacteria, in absence and presence of staining agent (Figures 11C to 11F). Difference in the contrasts in the SEM images of the sample surfaces also signifies the non-homogeneous chemical nature of the sample surface. Relatively brighter cell exterior for *Gram -ve* bacteria, as compared to the *Gram +ve* one indicates the accumulation of higher negative charge on the extra-cellular surface. This observation correlates well with the earlier reports which state that extra-cellular ATP release is higher for *Gram -ve* bacteria and its more porous surface with a protein called Porin. [(a) Wexler, H. M. Clinical Infectious Diseases. 1997, 25, S284. (b) Ivanova, E. P.; Alexeeva, Y. V.; Pham, D. K.; Nicolau, W. D.; Internationalmicrobiology, 2006, 9, 37. (c) Werner, T. P.; Amrhein, N.; Freimoser, F. M. BMC Plant Biology, 2007, 7:51 doi:10.1186/1471-2229-7-51.]

Staining of the *Yeast* and *bacterial* (*Gram* + ve) and (*Gram -ve*) cell using **L.Zn** (Scheme 1, Formula 1A) as

the staining agent and viewing stained cell culture using light microscope.

The isolates of *Saccharomyces sp.* were cultured in GYE (glucose yeast extract agar) media, *Bacillus sp.* in Nutrient broth, *and Pseudomonas sp.* in King's B medium for 24 hour at 37°C. 100 $\mu$l of this was taken and was allowed to incubate at room temperature along with 100 $\mu$l of [**L.Zn**] = 1.16 x $10^{-4}$M solution (pH 7.2, 10 mM HEPES buffer solution). The samples were fixed overnight at 25 to 35°C with 2 % (v/v) glutaraldehyde in 0.1 M phosphate buffer at pH 7.5. The samples were then; washed with 0.1 M phosphate buffer (pH 7.5) at 25 to 35°C for 1 hour. Post-fixation was carried out in 2 % (w/v) osmium tetroxide ($OsO_4$) in the same buffer and was washed once with 0.1 M phosphate buffer for 20 minutes. Then, the water was removed by a graded water-ethanol series; 25 % ethanol for 15 min, 50 % ethanol for 15min, 75 % ethanol for 30 min, 90 % ethanol for 60 min, absolute alcohol for 30 min. The specimens were rinsed in buffer and coated with gold in a sputter coater (Polaron SC7620) prior to microscopy. The material was examined in a scanning electron microscope (SEM) LEO 1430 VP at an accelerating Voltage of 15 kV.

**Enhancing the solubility of L.Zn (Scheme 1, Formula 1A) is aqueous solution (Scheme 2)**

Solubility of **L.Zn** (Scheme 1, Formula 1A) in water is limited (0.0448 $gL^{-1}$). However, this could be enhanced in presence of $\alpha$-cyclodextrin ($\alpha$-CD). 2.3 mg of **L.Zn** (Scheme 1, Formula 1A) was dissolved in 5 mL of double distilled water and stirred at 25 to 35°C for 1 hour. Then the solution was filtered and the concentration of the dissolved solid in the filtrate was evaluated based on the equation $O.D_{filtrate}/\varepsilon_{\mathbf{LZn}}$ ($O.D_{filtrate}$ is Optical Density of the filtrate and $\varepsilon_{\mathbf{LZn}}$ is the molar extinction coefficient of **L.Zn**) (value was evaluated earlier through an independent experiment) and was found to be 0.0448 $gL^{-1}$. Similar experiment was repeated in presence of $\alpha$-CD. In this case 14.2 mg of $\alpha$-CD was dissolved in 5 mL of double distilled water containing 2.913 mg of **L.Zn** (Scheme 1, Formula 1A) and this solution was stirred at 25°C for 1 hour; while a solution with little suspended solid was obtained. This was filtered using G-4 gooch crucible. Filtrate was collected and optical density (O.D.) of the filtrate was monitored at 458 nm. Considering the complete formation of the inclusion complex (pseudorotaxane form), concentration was evaluated based on the known $\varepsilon_{\mathbf{LZn.\alpha\text{-}CD}}$ value and the O.D. at 458 nm. Calculation revealed that 0.34 $gl^{-1}$ **L.Zn** (Scheme 1, Formula 1A) is present in the resultant solution. Hence ~ 7.5 fold increase in solubility in terms of **L.Zn** (Scheme 1, Formula 1A) was achieved and this is also evident in the difference of the visible color of the saturated solution of **L.Zn** (Scheme 1, Formula 1A) in absence and presence of $\alpha$-CD in aqueous solution (Figure 8). **L.Zn** (Scheme 1, Formula 1A) present in aqueous solution in the pseudorotaxane form is being represented as **L**.**Zn**.**CD** in Scheme 2.

**Characterisation of ligand L (Scheme 1, Formula 2)**

Isolated yield of the compound L (yield was calculated based on the starting compounds); 75 %. $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

**Characterization of L.Zn (Scheme 1, Formula 1A)**

Isolated yield of the compound **L.Zn** (yield was calculated based on the starting compounds); 65%. $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 - 2.74 (12H, br, $H_{h,i,j,l,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22. Analytical data agrees well with the proposed structure for this molecule.

**Evaluation of the stability constants for the inclusion complex formation between L.Zn (Scheme 1, Formula 1A) and $\alpha$-CD**

Preliminary spectral studies reveal that absorption maxima for **L.Zn** (Scheme 1, Formula 1A) (463 nm) tend to shift to shorter wavelength (458 nm) on addition of aqueous solution of $\alpha$-CD (0 - 2.3 mM) in excess to the 8.3 $\mu$M solution of **L.Zn** (Scheme 1, Formula 1A). Formation of host-guest type inclusion complex on addition of trans-1,2-bis-(4-pyridyl)ethylene, 4,4'-azopyridine (AZP) and some related bipyridine/biphenyl molecules in $\alpha$-CD is reported by several researcher (Shukla, A. D.; Bajaj, H. C.; Das, A. Angew. Chem. Int. Ed. 2001, 40, 446.; Shukla, A. D.; Bajaj, H. C., Das, A. Nat. Acad. Science, 2002, 114, 431.; Beer, A.J.; Macartney, D. H. Inorg. Chem. 2000, 39, 1410.; Macartney, D.H.; Waddling, C. A.; Inorg. Chem. 1994, 33, 5912.; Philip, D.; Stoddart, J.F. Angew. Chem. Int. Ed. 1996, 35, 1154.). It is also reported that inclusion in these compounds in the CD cavity influences the HOMO-LUMO energy gap and there by the absorption spectral bands-though the perturbation of the energy levels are not reported to be an appreciable one.

Thus, in this present study, the observed shift of 5 nm could be attributed to the inclusion phenomena and the formation of a pseudo-rotaxane complex. On systematic spectrophotometric titration absorption maxima was found to shift by 5 nm and two simultaneous isosbestic point was observed. Evaluation of the association constant based on this spectrophotometric titration revealed an association constant value of 255 (± 15) $M^{-1}$. General formula that was used for calculating the binding constants from UV-vis titrations is given below:

$$\mathbf{L.Zn} + \mathrm{CD} = \mathbf{L.Zn}.\mathrm{CD}$$

Absorption maxima for L is $\lambda_L$, while extinction coefficient and OD at this wavelength are $\varepsilon_L$ and $A_L$ respectively.

Absorption maxima for **L**.**Zn**.CD are $\lambda_{\mathbf{L.Zn.CD}}$ while extinction coefficient and OD at this wavelength are $\varepsilon_{\mathbf{L.Zn.CD}}$ and $A_{\mathbf{L.Zn.CD}}$ respectively.

Thus, for a given concentration of the receptor molecule (**[L]**) and α-cyclodextrin ([CD])

$$[\mathbf{L.Zn}.\mathrm{CD}] = [\{A_{\mathbf{L.Zn}.\mathrm{CD}} - A^0\} / \varepsilon_{\mathbf{L.Zn}.\mathrm{CD}}] \quad (1)$$

Where, $A^0$ is OD value at $\lambda_{\mathbf{L.Zn}.\mathrm{CD}}$ before addition of externally added CD.

$A_{\mathbf{L.Zn}.\mathrm{CD}}$ was recorded at $\lambda_{\mathbf{L.Zn}.\mathrm{CD}}$ after addition 100 mole equivalent of CD.

Therefore,

$$\lambda_{\mathbf{L.Zn}.\mathrm{CD}} = A_{\mathbf{L.Zn}.\mathrm{CD}} / [\mathbf{L.Zn}]_{free} \quad (2)$$

(Assuming all the receptor molecules is bound to CD and CD doesn't have any absorption at this wavelength)

Thus, once we one can evaluate [**L.Zn**.CD], it is possible to calculate concentration of the uncomplexed receptor molecule, [**L.Zn**] using eq. 1 and 3.

$$[\mathbf{L.Zn}]_{free} = [\mathbf{L.Zn}]_{initial} - [\mathbf{L.Zn}.\mathrm{CD}] \quad (3)$$

and,

$$[\mathrm{CD}]_{free} = [\mathrm{CD}]_{initial} - [\mathbf{L.Zn}.\mathrm{CD}] \quad (4)$$

If K is formation constant for the inclusion complex, **L.Zn.CD** for a given concentration of CD

Binding Constant,

$$K = [\mathbf{L.Zn}.\mathrm{CD}] / \{[\mathbf{L.Zn}]_{free}[\mathrm{CD}]_{free}\} \quad (5)$$

Thus, in presence of large excess of α-CD (125 fold), pseudorotaxane form, i.e., **L.Zn.α-CD** (Scheme 2) is expected to be present in predominant form in the aqueous solution (Figure 8).

Experiment to Determine Electronic Spectral Changes Attributable to the Coupling of the Compound, Represented as **L.Zn** (in Scheme 1, Formula 1A) with ATP and Other Anions were Performed.

In order to examine the binding behaviour of the Zn-complex (**L.Zn**) (Scheme 1, Formula 1A) to various important anions (like ATP, CTP, ADP, AMP, PPi, $H_2PO_4^-$, $SO_4^{2-}$, $CH_3CO_2^-$, $I^-$, $Br^-$, $Cl^-$ and $F^-$) the following experiment was conducted.

A solution (~ 21 mM) for of sodium salts of ATP, CTP, ADP, AMP, PPi, $H_2PO_4^-$, $SO_4^-$, $CH_3CO_2^-$, $I^-$, $Br^-$, $Cl^-$, and $F^-$ were prepared using respective salt in 10 mM HEPES buffer solutions (pH 7.2) and was.stored for use. Further, a solution (44.35 μM) of the compound **L.Zn** (Scheme 1, Formula 1A) was prepared using doubly-distilled and deionized water and stored. These stored solutions were used on the day of preparation of the solution for experiment.

A test solution was used after 1 mL of a probe storage solution (the previously prepared and stored solution of

44.35 μM for the compound (**L.Zn**) (Scheme 1, Formula 1A of the present invention) was put in a 10 mL test tube, such that the final concentration of the probe storage solution was 8.87 μM. To this 4 mL of each of the previous prepared and stored solutions of ATP and other anions (CTP, ADP, AMP, PPi, $H_2PO_4^-$, $SO_4^{2-}$, $CH_3CO_2^-$, $I^-$, $Br^-$, $Cl^-$ and $F^-$) was added to the test tube such that the aliquot of each of the solutions was (17 mM). Then the resultant solution was used for UV-vis spectral measurement after appropriate base line correction using a double beam spectrophotometer (Shimadzu UV-3101 PC) and the results thereof are shown in Figure 1.

As shown in Figure 1, in the absorption spectrum of the compound (**L.Zn**) (Scheme 1, Formula 1A), according to the present invention, when ATP was added at pH 7.2, a distinctly new absorption spectral band at 503 nm developed. Thus a red shift of 40 nm was observed on addition of the solution of ATP (~ 1920 equiv.) to the solution of **L.Zn** (Scheme 1, Formula 1A). This shift for CTP was much less significant and a red shift of only 9 nm was observed. In contrast, when 2000 equivalents of other anions were added, no change was observed. Therefore, it can be seen that the compound (**L.Zn**) (Scheme 1, Formula 1A), according to the present invention, is preferentially coupled with ATP.

A solution of **L.Zn** (Scheme 1, Formula 1 A) (8.87 μM) in aqueous medium was treated with excess amount of ~ 500 μM of different phosphate anions like $H_2PO_4^-$, PPi, AMP, ADP, ATP and CTP. On addition of the respective anion, significant color change was only observed in presence of ATP. Slight color change was observed in case of ADP and CTP. Thus **L.Zn** (Scheme 1, Formula 1A) can bind ATP selectively among various phosphate anions and the color change associated with the binding of ATP can be detected through naked eye (Figure 2).

Uv-vis Spectral Titration Experiment on the Compound (**L.Zn**) (Scheme 1, Formula 1A) Depending on the concentration of ATP.

In order to examine the binding characteristics between the Zn(II)-compound (**L.Zn**) (Scheme 1, Formula 1A) and ATP, the following experiment was conducted.

A solution of ATP (21.6 mM) was prepared using a 10 mM N-2-Hydroxyethylpiperazine-N'-2-ethanesulfonic acid (HEPES) buffer solution (pH 7.2). Further, a solution (47.3 μM)) of the compound (**L.Zn**) (Scheme 1, Formula 1A, obtained in Example 1) was prepared using doubly-distilled and deionized water. These storage solutions were used on the day of preparation of the solution for experiment.

A 1.0 mL of stored solution of **L.Zn** (Scheme 1, Formula 1A) ([**L.Zn**] = 47.3 μM) was put in a test-tube, such that, the final concentration of the **L.Zn** solution was 9.46 μM. To this varying amount of ATP (0.1 - 4 mL of the stored ATP solution (21.6 mM) was added. Resulting solution was then diluted by addition of a 10 mM HEPES buffer solution (pH 7.2) maintaining total volume of the resultant aliquot of 5mL, which was used for spectrophotometric measurements using a double beam spectrophotometer and quartz spectrophotometric cell (3.5 mL capacity). The total spectral change was measured using a double beam spectrophotometer (Shimadzu UV-3101 PC) after appropriate base line correction. Slit width for all experiments during this titration was kept unchanged at 0.2 nm and the results thereof are shown in Figure 3.

As shown in Figure 3, in the absorption titration spectrum of the compound **L.Zn,** (Scheme 1, Formula 1A) with varying ATP. According to the present invention, when the final concentration of the compound was 9.46 μM at pH 7.2, the amount of added ATP was increased from 0 equivalents to 1826 mole equivalents, and thus a new spectral band due to the formation of the complex **L.Zn-ATP** (Scheme 1, Formula 1B) was observed at a wavelength of 503 nm. Therefore, it can be seen that the binding constant for the formation of the complex (**L.Zn-ATP**) (Scheme 1, Formula 1B) of the present invention was 978 ± 15 $M^{-1}$ (25°C) through the spectrophotometric titration experiment of the compound and ATP.

General formula used for calculating the binding constants from UV-vis titrations.

$$\mathbf{L.Zn} + A^- = \mathbf{L.Zn\text{-}A^-}$$

An absorption maximum for L.Zn is $\lambda_L$, while extinction coefficient and OD at this wavelength are $\varepsilon_L$ and $A_L$ respectively.

An absorption maximum for **L.Zn-A**$^-$ is $\lambda_{L.Zn\text{-}A^-}$ while extinction coefficient and OD at this wavelength are $\varepsilon_{L.Zn\text{-}A^-}$ and $A_{L.Zn\text{-}A^-}$, respectively.

Thus, for a given concentration of the receptor molecule ([**L.Zn**]) and anion ([$A^-$])

$$[\mathbf{L.Zn\text{-}A^-}] = [\{A_{LZn\text{-}A^-} - A^0\} / \varepsilon_{L.Zn\text{-}A^-}] \qquad (6)$$

Where, $A^0$ is OD value at $\lambda_{L.Zn\text{-}A^-}$ before addition of externally added A .

$A_{L.Zn\text{-}A^-}$ was recorded at $\lambda_{L.Zn\text{-}A^-}$ after addition 100 mole equivalent of $A^-$.

Therefore,

$$\varepsilon_{L.Zn\text{-}A^-} = A_{L.Zn\text{-}A^-} \;/\; [\mathbf{L.Zn}]_{free} \qquad (7)$$

(Assuming all the receptor molecules is bound to $A^-$ and $A^-$ alone do not absorb at this wavelength)

Thus, once we one can evaluate [**L.Zn-A**$^-$], it is possible to calculate concentration of the uncomplexed receptor molecule, [L.Zn] using eq. 6 and 7.

$$[\mathbf{L.Zn}]_{free} = [\mathbf{L.Zn}]_{initial} - [\mathbf{L.Zn\text{-}A^-}] \qquad (8)$$

and,

$$[A^-]_{free} = [A^-]_{initial} - [\mathbf{L.Zn\text{-}A^-}] \qquad (9)$$

If K is the formation constant for the inclusion complex, **L.Zn-A**$^-$ for a given concentration of $A^-$

Binding Constant,

$$K = [\mathbf{L.Zn\text{-}A^-}] \;/\; \{[\mathbf{L.Zn}]_{free}[A^-]_{free}\} \qquad (10)$$

A 1.0 mL of stored solution **L.Zn** (Scheme 1, Formula 1A) ([**L.Zn**] = 8.95 μM) was put in a test-tube, such that, the final concentration of the **L.Zn** solution was 1.79 μM to this varying amount of CTP (0.1-4 mL of the stored CTP solution (12.71 mM) was added. Resulting solution was then diluted by addition of a 10 mM HEPES buffer solution (N-(2-hydroxyethyl)-piperazine-N'-2-ethanesulfonic acid) (pH 7.2) maintaining total volume of the resultant aliquot of 5 mL, which was used for spectrophotometric measurements by putting a quartz spectrophotometric cell (3.5 mL capacity). The total spectral change was measured using a spectrophotometer (Shimadzu UV-3101 PC) after appropriate base line correction. Slit width for all experiments during this titration was kept unchanged at 0.2 nm and the results thereof are shown in Figure 4.

As shown in Figure 4, in the absorption titration spectrum of the compound **L.Zn,** (Scheme 1, Formula 1A) with varying CTP. According to the present invention, when the final concentration of the compound was 1.79 μM at pH 7.2, the amount of added CTP was increased from 0 equivalents to 5681 equivalents, and thus a new spectral band due to the formation of the complex **L.Zn-CTP** was observed at a wavelength of 477 nm. Therefore, it can be seen that the binding constant for the formation of the complex (**L.Zn-CTP**) of the present invention was 220 (±15) $M^{-1}$ (25°C), through the spectrophotometric titration experiment of the compound and CTP.

A 1 mL of stored solution **L.Zn** (Scheme 1, Formula 1A) ([**L.Zn**] = 44.3 μM) was put in a test-tube, such that, the final concentration of the **L.Zn** solution was 8.86 μM. To this varying amount of ADP (0.1-4 mL of the stored ADP solution (8.19 mM) was added. Resulting solution was then diluted by addition of a 10 mM HEPES buffer solution (pH 7.2) maintaining total volume of the resultant aliquot of 5mL, which was used for spectrophotometric measurements using a matched pair of quartz spectrophotometric cell (3.5 mL capacity). The total spectral change was measured using a double beam spectrophotometer (Shimadzu UV-3101 PC) after appropriate base line correction. Slit width for all experiments during this titration was kept unchanged at 0.2 nm and the results thereof are shown in Figure 5.

As shown in Figure 5, in the absorption titration spectrum of the compound **L.Zn,** (Scheme 1, Formula 1A) with varying ADP. According to the present invention, when the final concentration of the compound was 8.86 μM at pH 7.2, the amount of added ADP was gradually increased from 0 to 924 mole equivalents, and thus a new spectral band due to the formation of the complex **L.Zn-ADP** was observed at a wavelength of 471 nm. Therefore, it can be seen that the binding constant for the formation of the complex **(L.Zn-ADP)** of the present invention was 142 (±15) $M^{-1}$ (25°C), through the spectrophotometric titration experiment of the compound and ADP.

$^{31}P$ NMR spectra of ATP and CTP (Figure 6A and Figure 6B) were recorded on Bruker 500 MHz (Bruker Avance II-DPX 500 MHz) FT NMR. Binding of ATP with **L.Zn** (Scheme 1, Formula 1A) was also confirmed by $^{31}P$ NMR spectroscopy (Figure 6A). Up field shifts for the $^{31}P$ signals for the α, β and γ-P-atoms (0.07 ppm, 0.09 ppm and 0.05 ppm, respectively) of the ATP bound to **L.Zn** (Scheme 1, Formula 1A) were observed. Almost similar shifts have been reported earlier by others. (Ojida, A.; Park, S. K.; Mito-oka, Y.; Hamachi, I. Tetrahedron Lett. 2002, 43, 6193). The $^{31}P$ NMR

signals for α, β and γ-P atoms signify the binding to Zn-atom of **L.Zn** (Scheme 1, Formula 1A) through the respective phosphate units: This observation leads us to conclude that **L.Zn** (Scheme 1, Formula 1A) is capable of forming hepta coordinated complex in presence of ATP. Example of this type of hepta coordinated Zn complex are there in the literature [(a) Lindloy, L. F.; Busch, D. H. Inorg. Chem. 1974, 13, 2494. (b) Wester, D.; Palenik, G. J. J. Am. Chem. Soc. 1974, 95, 6505.]

A very insignificant shift in $^{31}P$ signals were observed when similar experiments were repeated for CTP (Figure 6B). Up feld shifts for the $^{31}P$ signals for the α, β and γ-P-atoms (0.0 ppm, 0.03 ppm and 0.01 ppm, respectively) of the CTP, bound to **L.Zn** (Scheme 1, Formula 1A), were observed. No such shifts were observed when identical experiments were performed with ADP and AMP. Thus, the $^{31}P$ NMR spectral data also confirms the observed trend in binding affinity (ATP >> CTP > ADP >> AMP). Presumably, the enhanced electrostatic interaction between ATP and **L.Zn** (Scheme 1, Formula 1A) is crucial for efficient **L.Zn**-O(phosphate) binding in **L.Zn-ATP.** Hence, the observed binding preference of **L.Zn** (Scheme 1, Formula 1A), ATP >> CTP > ADP >> AMP, could be attributed to the difference in the number of the anionic charges of the phosphate species.

Mass Analysis of a Complex **L.Zn** (Scheme 1, Formula 1A) in Absence and Presence of ATP were Performed Using Electronspray Ionization (ESI) Technique.

In order to evaluate whether, or not a complex of the compound (**L.Zn,** Scheme 1, Formula 1A) according to the present invention and ATP was formed, electron spray ionization mass analysis was conducted with reference to the document (Kwon, J. Y.; Jang. Y. J.; Lee, Y. J.; Kim, K. M.; Seo M. S.; Nam, W.; Yoon, J. J. Am. Chem. Soc. 2005, 127, 10107).

The compound **L.Zn** (Scheme 1, Formula 1A; 7.2 μM) and 900 μM of ATP (125 mole equivalent) were put into volumetric flask and dissolved in 5 mL of HPLC grade water to form a mixed solution. Then this mixed solution was mass-analyzed using electronspray ionization (ESI) technique in positive mode. As a result, peaks were obtained around m/z = 1082 and is shown in Figure 7 (instrument used: Thermo Finnigan LCQ™ Advantage MAX quadrupole ion trap instrument, San Jose, Calif., USA) (Figure 7).

On addition of the aqueous solution of the disodium salt of ATP to the aqueous solution of **L.Zn** (Scheme 1, Formula 1A), a new complex **L.Zn-ATP** (Scheme 1, Formula 1B) was formed. This solution was used for recording the ESI-Mass spectra and the spectrum is shown in figure 7. Peaks, shown in Figure 7, indicate masses corresponding to $[C_{34}H_{49}N_{12}P_3SO_{15}NaZn + H^+] = [[C_{24}H_{37}N_7P_3SO_2Zn]$ of $L.Zn(NO_3)_2 - 2(NO_3^-)] + [C_{10}H_{12}N_5O_{13}P_3Na_2 - Na^+] + H^+$. It is evident that it supports the formation of a 1:1 complex formation between **L.Zn** (Scheme 1, Formula 1A) and ATP. Therefore, mass spectra analysis corroborates formation of the proposed complex, **L.Zn-ATP**.

As described above in the discussion on the electronic spectral studies, the Zn(II) complex, **L.Zn** (Scheme 1, Formula 1A) of the present invention does not recognize other anions in aqueous solution, and has shown selectivity for ATP. On binding to the ATP, a new absorption band appeared at 503 nm for **L.Zn** (Scheme 1, Formula 1A) owing to a more preferred charge transfer transition with a distinct color change that could be detected in naked eyes. Accordingly, this offered us the opportunity to explore the possibility of using this Zn(II)-complex (**L.Zn,** Scheme 1, Formula 1A) of the present invention as colorimetric staining agent for living cells for viewing simply through light microscope.

*Saccharomyces cerevisiae (S. cerevisiae),* a Eukaryotic microbe, derive their energy in the form of ATP and the surface of these cells possesses negatively charged ATP ions. We have therefore, been able to use the receptor molecule, **L.Zn** (Scheme 1, Formula 1A) for staining Eukaryotic cells. *Yeast* cells were exposed to the receptor molecule **L.Zn** (Scheme 1, Formula 1A) ($1.16 \times 10^{-4}$ M) for 20 min or **L.Zn-CD** (Scheme 2) (([**L.Zn**] = $0.5 \times 10^{-3}$ M [α-**CD**] = $2.92 \times 10^{-3}$ M) for 2 min followed by subsequent washing with water mixtures and then viewed under a light microscope (AXIO IMAGER-Carl Zeiss).

Preliminary spectral studies reveal that absorption maxima for **L.Zn** (Scheme 1, Formula 1A) (463 nm) shifts to shorter wavelength (458 nm) on addition of aqueous solution of α-CD (0 - 2.3 mM) to the 8.3 μM solution of **L.Zn** (Scheme 1, Formula 1A). Inclusion in the CD cavity is expected to influence the HOMO-LUMO energy gap and there by the absorption spectral bands-though the perturbation of the energy levels are not expected to be an appreciable one. Thus, in this present study, the observed shift of 5 nm could be attributed to the inclusion phenomena and the formation of a pseudo-rotaxane complex. On systematic spectrophotometric titration, absorption maximum was found to shift by 5 nm and two simultaneous isosbestic points was observed. Evaluation of the association constant, based on this spectrophotometric titration, revealed an association constant value of 255 (±15) $M^{-1}$. Thus, in presence of large excess of α-CD (125 fold), pseudorotaxane form, i.e., **L.Zn.α-CD** (Scheme 2) is expected to be present in predominant form in the aqueous solution (Figure 8).

The images of the colorless *Yeast* cells, viewed under normal light microscopy (AXIO IMAGER-Carl Zeiss), are shown in Figure 9A. Images of the treated cells with **L.Zn** (Scheme 1, Formula 1A) (0.116 mM) and **L.Zn.α-CD** (Scheme 2) (([**L.Zn**] = 0.5 mM, [α-**CD**] = 2.92 mM) thus observed are shown in figures 9B and 9C (with similar magnification), respectively. Figure 9A revealed that treated cell became stained with **L.Zn** (Scheme 1, Formula 1A) and that the color of the cells changed to pink-violet (Figure 9A(B)). The change in color of *Yeast* cells indicated that the negatively charged phosphate groups on the surface of the cells were effectively bound to the receptor molecule **L.Zn** (Scheme 1,

Formula 1A). The staining was found to be stable even after subsequent washing with water/ethanol (70/30, v/v) mixtures. Ethanol is known to act as a dehydrator and a good fixative of biological samples. Control experiments were performed by treating these cells with **L.Zn** (Scheme 1, Formula 1A) or **L.Zn.**$\alpha$**-CD** (Scheme 2) or a solution of free Zn(II) ions; while no such change in color was observed. This confirms the binding of the ATP ions of the cell surface to **L.Zn** (Scheme 1, Formula 1A) as the plausible reason for the observed staining. Binding of the ATP ions, produced *in situ,* on the cell surface to **L.Zn** (Scheme 1, Formula 1A) was accounted as the plausible reason for the observed staining. Further absorption spectra (Figure 10A) and light microscopic images (Figure 10B) revealed that the stained cells became colorless on addition of excess (50 mole equivalent) of citrate ion to the ATP as well as to the stained *Yeast* cell. Same observation was observed in the mixture of *Yeast* cells and **L.Zn/ L.Zn.**$\alpha$**-CD** (Scheme 2) (Figure 10B). This observation could be explained on the basis of the reversible binding of ATP, produced *in situ* in the *Yeast* cells.

We also explored the possibility of using **L.Zn/ L.Zn.**$\alpha$**-CD** as the staining agent for *prokaryotes (Gram +ve* and *Gram -ve* bacterial cells). Figures 9B(A) and 9C(A) revealed the images of the two bacteria, *Bacillus megaterium* (*Gram +ve*) and *Pseudomonas fluorescence* (*Gram -ve*), respectively, when viewed under light microscope. Figure 9B(B) and 9C(B) revealed images of same bacteria when viewed in presence of **L.Zn** (Scheme 1, Formula 1A) (66 $\mu$M). These figures clearly showed a distinct change in color of the bacterial stains in presence of **L.Zn**. Figures 9b(C) and 9c(C) revealed images of same bacteria when viewed in presence of **L.Zn.**$\alpha$**-CD** ([**L.Zn**]=**0.5** mM, [$\alpha$-**CD**] = 2.92 mM). Figures 9B and 9C, taken with identical magnification, revealed that present dye **L.Zn** (Scheme 1, Formula 1A) could also distinguish *Gram +ve* and *Gram -ve* bacteria through distinctly different color intensities and shape of the stained cells. As expected stained cells for *Gram +ve* bacteria appeared longer as compared to those of the stained *Gram -ve* bacteria. The difference in the staining intensity for two different bacteria with the **L.Zn.**$\alpha$**-CD** (Scheme 2) could be much better understood after going into the depth of cell biology of each bacterial cells and the detailed biochemistry involved while staining; besides, difference in cell structure and cell wall composition of the respective bacteria.

SEM images (Figure 11) of blank and stained eukaryote (*Yeast*), *Gram +ve* and *Gram - ve* cells revealed the change(s) in the morphology of the outer surface of the cells on staining. SEM images of *Yeast* cells without dye were found to be smooth in contrast to the images of the *Yeast* cells with dye, where stained cell surfaces were found to be rough. This revealed the presence of the extraneous material i.e **L.Zn** (Scheme 1, Formula 1 A) adhering to the cell. Changes on the cell surfaces on binding of the dye, **L.Zn** (Scheme 1, Formula 1A) were also evident from the SEM images recorded for *Gram -ve* and *Gram +ve* bacteria, in absence and presence of staining agent (Figures 11C to 11F). Difference in the contrasts in the SEM images of the sample surfaces also signifies the non-homogeneous chemical nature of the sample surface. Relatively brighter cell exterior for *Gram -ve* bacteria, as compared to the *Gram +ve* one indicates the accumulation of higher negative charge on the extra-cellular surface.

Unaffected cell proliferation and growth confirmed that the staining agent (either **L.Zn** or **L.Zn.**$\alpha$**-CD**) was non-toxic and kept cells viable after staining. Thus; **L.Zn** (Scheme 1, Formula 1A) could be used as an efficient *in-situ* chemosensor for bioactive molecules like ATP and also as a viable staining agent. The growth profile of Eukaryotic *Yeast* cells (*Saccharomyces cerevisiae*) and Prokaryotic bacterial cells (*Gram +ve* (*Pseudomonas fluorescence*) and *Gram -ve (Bacillus megaterium)*) in an aqueous culture medium in absence and presence of L.Zn (Scheme 1, Formula 1A) are shown in Figure 12. Change in absorbance (at $\lambda_{max}$ = 600 nm) *vs* time (t) was plotted up to 30 h; as no appreciable change in growth was observed thereafter. Figures 12a-f revealed a nice correlation between the cell growth and the ATP generation with progressive growth of the respective cells during the metabolic processes. These kinetic studies were carried out to monitor the different phases of growth; wherein, the increase in the ATP concentration as the batch culture grows from lag phase to log phase and then stationary phase and ultimately a decline in the growth curve was observed. Figures 12a, 12c and 12e show the actual growth curve of *Saccharomyces cerevisiae, Pseudomonas fluorescence* and *Bacillus megaterium,* respectively with time. While, Figures 12b, 12d and 12f show relative build up of ATP in cytoplasm with time for *Saccharomyces cerevisiae, Pseudomonas fluorescence* and *Bacillus megaterium,* respectively in presence of **L.Zn** (Scheme 1, Formula 1A) (D1) and **L.Zn.**$\alpha$**-CD** (Scheme 2) (D2). Figure 12 reveals that the actual cell growth and buildup in ATP concentration in the cultures under identical experimental conditions matched nicely. The relative decrease, observed in actual cell growth and ATP concentration (Figures 12) can be explained if one considers the catabolic processes, which consume ATP, become more important after certain time.

Neither ATP, nor ADP/AMP/PPi can be stored at high concentration within the cells of the living system. Their relative concentrations in cytoplasm are generally regulated over a narrow concentration limit by various biological processes. Actually, bacteria impose regulatory mechanisms on metabolic processes to ensure that, the needs of the cell are met but not exceeded. Thus, the physiological consequences of this regulation are the same, though the mechanism in *Gram +ve* and *Gram -ve* bacteria is entirely different. However; kinetic studies revealed that cell growth and release of ATP during metabolic process could be correlated using the **L.Zn** (Scheme 1, Formula 1A) as the staining agent. Extent of absorbance changes and the higher slope observed in case of *Gram -ve* bacteria revealed a higher binding of **L.Zn** (Scheme 1, Formula 1A) to ATP released during the cell growth, as compared to *Gram +ve.*

The viability of the cells was observed before and after staining with the **L.Zn** (Scheme 1, Formula 1A) under the light microscope. The cell growth, cell division, as well as their motility could be observed when the stained cell

suspension was taken as a hanging drop prepared in a concavity slide until 3 h (Figure 13).

We have synthesized a new Zn(II) based colorimetric sensor **L.Zn** (Scheme 1, Formula 1A) following easy two step synthetic procedure, where the metal center acts as receptor unit for binding with anionic analytes. The sensor is selective for ATP in pure aqueous medium and shows significant color change after binding to ATP. Ability of this compound for live cell staining through binding to ATP, produced in different biological cells (Eukaryotic and Prokaryotic cells) was examined. This reagent was found to stain cells, which could be viewed through light microscope with naked eyes and was found to be non-toxic. This viable staining agent allowed the study on cell growth dynamics.

The present invention deals with the preparation of Zn(II) based colorimetric sensor **L.Zn** (Scheme 1, Formula 1A) and developing the process for its use as a viable staining agent for living cells. Here the preparation of sensor involves the synthesis of ligand and its reaction with zinc salt solution. The metal centre acts as a receptor unit for binding with anionic analytes. The sensor is selective for adenosine triphosphate (ATP) in aqueous medium and evinces significant color change after binding with ATP. The sensor functions as staining agent and the study on cell growth dynamics could be undertaken. The prior art neither divulge nor teach how the Zn(II)-based colorimetric sensors could be used as viable staining agent for living cell. It is reported for the first time in this invention that *Yeast* cells (Eukaryotic) and *Gram +ve* / *Gram* - ve bacterial cells (Prokaryotic) can be differentiated based on the colour intensity of the stained cell. Moreover, the sensor is non toxic and can be used for evaluation and monitoring of the cell growth dynamics of biological cell in aqueous medium even after staining of cells.

The inventive steps adopted in the present invention are (i) preparation of Zn(II) based colorimetric sensor **L.Zn** (Scheme 1, Formula 1A), which preferentially binds ATP among various biological phosphates, (ii) the sensor is active for staining living cells under physiological conditions at pH 7.2 in aqueous medium; (iii) the sensor is non toxic for the living cells and (iv) inclusion of sensor into $\alpha$-cyclodextrin increases the staining intensity of living cell and subsequent separation based on colour intensity. The technical problem that we have solved through this invention is developing a viable staining agent for live cell that could be used for evaluation of the *in vivo* cell growth dynamics through recognition of variable concentration of ATP produced during the respiratory process. One of the major problem that is encountered in the hitherto known process for sensors, useful for staining living cells in physiological condition is the toxicity of the sensors. Toxicity of these sensors used does not allow studying the *in vivo* cell growth dynamics as these cause death of living cells. In contrast, the Zn-based colorimetric sensor of the present invention was not toxic and found to be useful for evaluation of the *in vivo* cell growth dynamics of the biological cells. Besides, with the help of this sensor a correlation between cell growth and the *in situ* adenosine triphosphate (ATP) generation with progressive growth of the respective cell during the metabolic processes could be established.

## EXAMPLES

The following examples are given by way of illustration and therefore should not be construed to limit the scope of the present invention.

### Example 1

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5 mL of triethylamine was added at 1.0 mL/min. A separately prepared solution containing 400 mg of 4-(4'-dimethylamino phenyl azo) benzene sulphonyl chloride in 50 mL tetrahydrofuran (24.76 mM concentration) was added at 50 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.5 mole equivalent of the ligand was added at a temperature of 25°C and kept stirring for a period of 15 hours. The temperature of resulting mixture was lowered to 15°C by freeze cooling and further agitates for another 3 hours. The precipitate of the desired compound (sensor) was washed with chloroform and kept aside. 100 $\mu$L of the *Saccharamyces* species in GYE media was incubated with 100 $\mu$L of sensor at a concentration of $1.0 \times 10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield; 75 % (Yield was calculated based on the starting compounds). $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A): Yield; 65 % (Yield was calculated based on the starting compounds). $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d,

J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ve mode, m/z): 676.00 ($M^+$). Elemental Analysis: Calc. C: 42.57, H: 5.51, N: 18.62; Expt. C: 42.88, H: 5.56, N: 18.22.

## Example 2

In a 250 mL capacity round bottom flask, 247 mg (1.23 mM concentration) of 1,4,8,11 tetraazacyclotetradecane (cyclam) was dissolved in 154 mL dry tetrahydrofuran (8.0 mM concentration) under mild stirring at 25°C. The flask with the solution was kept in ice at a temperature of 5°C was maintained. To this 5 mL of triethylamine was added at 1.0 mL/min. A separately prepared solution containing 400mg 4-(4'-dimethylamino phenyl azo) benzene sulphonyl chloride in 50 mL tetrahydrofuran (24.76 mM concentration) was added at 50 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.5 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with chloroform and kept aside. 100 $\mu$L of the *Saccharamyces* species in GYE media was incubated with 100 $\mu$L of sensor at a concentration of $1.0x10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield; 70 % (Yield was calculated based on the starting compounds). $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A): Yield; 65% (Yield was calculated based on the starting compounds); $^1$HNMR (500 MHz, DMF-$d_7$, TMS, $\delta$ (ppm)): 7.92 - 7.911 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22.

## Example 3

In a 250 mL capacity round bottom flask, 247 mg (1.23 mM concentration) of 1,4,8,11 tetraazacyclotetradecane (cyclam) was dissolved in 100 mL dry dichloromethane (12.3 mM concentration) under mild stirring at 25°C. The flask with the solution was kept in ice at a temperature of 5°C was maintained. To this 5 mL of triethylamine was added at 1.0 mL/min. A separately prepared solution containing 400 mg 4-(4'-dimethylamino phenyl azo) benzene sulphonyl chloride in 50 mL dry dichloromethane (24.76 mM concentration) was added at 50 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.5 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with chloroform and kept aside. 100 $\mu$L of the *Saccharamyces* species in GYE media was incubated with 100 $\mu$L of sensor at a concentration of $1.0x10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield; 30% (Yield was calculated based on the starting compounds). $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A) : Yield; 65% (Yield was calculated based on the starting compounds). $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$). ES-ms (+ ve mode, m/z): 676.00 ($M^+$). Elemental Analysis: Calc. C: 42.57, H: 5.51, N: 18.62; Expt. C: 42.88, H: 5.56, N: 18.22.

**Example 4**

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5 mL of triethylamine was added at 1.0 mL/min. A separately prepared solution containing 400 mg 4-(4'-dimethylamino phenyl azo) benzene sulphonyl chloride in 62 mL tetrahydrofuran (20 mM concentration) was added at 50 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.5 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with chloroform and kept aside. 100 $\mu$L of the *Saccharamyces* species in GYE media was incubated with 100 $\mu$L of sensor at a concentration of $1.0 x 10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield 68% (Yield was calculated based on the starting compounds). $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A): Yield 65% (Yield was calculated based on the starting compounds). $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22.

**Example 5**

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5 mL of triethylamine was added at 1.0 mL/min. A separately prepared solution containing 400 mg 4-(4'-dimethylamino phenyl azo) benzene sulphonyl chloride in 62 mL tetrahydrofuran (20 mM concentration) was added at 25 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.35 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with chloroform and kept aside. 100 $\mu$L of the *Saccharamyces* species in GYE media was incubated with 100 $\mu$L of sensor at a concentration of $1.0 x 10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield 75% (Yield was calculated based on the starting compounds). $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A): Yield 50% (Yield was calculated based on the starting compounds). $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22.

**Example 6**

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5 mL of triethylamine was added at 1.0 mL/min. A separately prepared solution containing 400 mg 4-(4'-dimethylamino phenyl azo) benzene sulphonyl

chloride in 62 mL tetrahydrofuran (20 mM concentration) was added at 25 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.75 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with chloroform and kept aside. 100 $\mu$L of the *Saccharamyces* species in GYE media was incubated with 100 $\mu$L of sensor at a concentration of 1.0x10$^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield 75% (Yield was calculated based on the starting compounds). $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A): Yield 63% (Yield was calculated based on the starting compounds). $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22.

**Example 7**

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5 mL of triethylamine was added at 1.0 mL/min. A separately prepared solution containing 400mg 4-(4'-dimethylamino phenyl azo) benzene sulphonyl chloride in 62 mL tetrahydrofuran (20 mM. concentration) was added at 25 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.75 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with chloroform and kept aside. 100 $\mu$L of the *Bacillus* species in Nutrient broth media was incubated with 100 $\mu$L of sensor at a concentration of 1.0x10$^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield 75% (Yield was calculated based on the starting compounds). $^1$H NMR (500 MHz, $CDCl_3$, TMS, J (Hz), $\delta$ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A): Yield 65% (Yield was calculated based on the starting compounds). $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22.

**Example 8**

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5 mL of triethylamine was added at 1.0 mL/min. A separately prepared solution containing 400 mg 4-(4'-dimethylamino phenyl azo) benzene sulphonyl chloride in 62 mL tetrahydrofuran (20 mM concentration) was added at 25 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15mM. To this Zinc nitrate solution of 0.75 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with

chloroform and kept aside. 100 μL of the *Pseudomonas* species in King's B media was incubated with 100 μL of sensor at a concentration of 1.0x$10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield 75% (Yield was calculated based on the starting compounds). $^1$H NMR (500 MHz, $CDCl_3$, TMS, J (Hz), δ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Isolated yield of the compound **L.Zn** (Scheme 1, Formula 1A): Yield 65% (Yield was calculated based on the starting compounds). $^1$HNMR (500 MHz, DMF-$d_7$, TMS, δ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22.

## Example 9

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5 mL of triethylamine was added at 1.0 mL/min. A separately prepared solution containing 400 mg 4-(4'-dimethylamino phenyl azo) benzene sulphonyl chloride in 62 mL tetrahydrofuran (20 mM concentration) was added at 25 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15mM. To this Zinc nitrate solution of 0.75 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) of the desired compound (**L.Zn,** (Scheme 1, Formula 1A)) was washed with chloroform and kept aside. 100 μL of the *Pseudomonas* species in King's B media was incubated with 100 μL of sensor at a concentration of 1.25 x$10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield 75% (Yield was calculated based on the starting compounds). $^1$H NMR (500 MHz, $CDCl_3$, TMS, J (Hz), δ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$), 2.03 (4H, br, $H_{g,l}$). Es-Ms (+ ve mode): m/z; 488.30 ($M^+$+$H^+$). Elemental Analysis data: Calc. C: 59.11, H: 7.65, N: 20.11, Expt. C: 59.1, H: 7.5, N: 20.2.

Yield 65% (Yield was calculated based on the starting compounds). $^1$HNMR (500 MHz, DMF-$d_7$, TMS, δ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -$N(CH_3)_2$), 2.92-2.91 (8H, br, $H_{f,g,n,o}$), 2.75 -2.74 (12H, br, $H_{h,i,j,k,l,m}$),. ES-ms (+ ve mode, m/z): 676.00 ($M^+$). Elemental Analysis: Calc. C: 42.57, H: 5.51, N: 18.62; Expt. C: 42.88, H: 5.56, N: 18.22.

## Example 10

In a 250 mL capacity round bottom flask, 247 mg (1.23 mM concentration) of 1,4,8,11 tetraazacyclotetradecane (cyclam) was dissolved in 100 mL dry tetrahydrofuran (12.3 mM concentration) under mild stirring at 25°C. The flask with the solution was kept in ice at a temperature of 5°C was maintained. To this 5mL of triethylamine was added at 1.0 mL/min. A separately prepared solution containing 400 mg 4-(4'-dimethylamino phenyl azo) benzene sulphonyl chloride in 62 mL tetrahydrofuran (20 mM concentration) was added at 25 mL/hour under stirring and at temperature of 5°C. The mixture after attaining the temperature of 25°C was stirred for 6 hours and then refluxed for 30 minutes. The filtered and washed precipitate (ligand) was dissolved in methanol at 25°C to have a concentration of 15 mM. To this Zinc nitrate solution of 0.75 mole equivalent of the ligand was added at a temperature of 25°C and kept stirring for a period of 15 hours. The temperature of resulting mixture was lowered to 15°C by freeze cooling and further agitates for another 3 hours. The precipitate (sensor) of the desired compound (**L.Zn,** (Scheme 1, Formula 1A)) was washed with chloroform and kept aside. 100 μL of the *Pseudomonas* species in King's B media was incubated with 100 μL of sensor at a concentration of 1.45 x$10^{-4}$ M at 25°C for a period of 10 mins. Based on different color intensity the *Gram +ve* and *Gram -ve* were distinguished and colonies of the test microbes grew when streaked on the nutrient agar plates.

Isolated yield of the compound **L** (Scheme 1, Formula 2): Yield 75% (Yield was calculated based on the starting compounds). $^1$H NMR (500 MHz, $CDCl_3$, TMS, J (Hz), δ (ppm)): 7.98 - 7.96 (2H, d, J = 8.0, Ar-$H_e$) 7.9 - 7.87 (4H, m, Ar-$H_c$,$H_d$), 7.06 (3H, b, -$NH_{macrocycle}$), 6.77-6.75 (2H, d, J = 8.0, Ar-$H_b$), 3.15 (16H, br, $H_{f,h,i,j,k,m,n,o}$), 3.11 (6H, s, -$N(CH_3)_2$),

2.03 (4H, br, $H_{g,l}$). Es-Ms (+ ve mode): m/z; 488.30 ($M^++H^+$). Elemental Analysis data: Calc. C: 59.11, H: 7.65, N: 20.11, Expt. C: 59.1, H: 7.5, N: 20.2.

Yield 65% (Yield was calculated based on the starting compounds). $^1$HNMR (500 MHz, DMF-$d_7$, TMS, $\delta$ (ppm)): 7.92 - 7.91 (2H, d, J = 8.5, Ar-$H_e$), 7.88 - 7.86 (2H, d, J = 9, Ar-$H_c$), 7.81 -7.79 (2H, d, J = 8.5, Ar-$H_d$), 6.92 - 6.9 (2H, d, J = 9.5, Ar-$H_b$), 3.13 (6H, s, -N($CH_3$)$_2$), 2.92-2.91 (8H, br, $H_{f,g,n,o}$), 2.75 -2.74 (12H, br, $H_{h,i,j,k,l,m}$),. ES-ms (+ve mode, m/z): 676.00 ($M^+$). Elemental Analysis: Calc. C: 42.57, H: 5.51, N: 18.62; Expt. C: 42.88, H: 5.56, N: 18.22.

## ADVANTAGES OF THE INVENTION

1. The Zn (II) based colorimetric sensor preferentially binds with Adenosine triphosphate (ATP) than the other nucleotides.
2. The sensor is nontoxic to living cell.
3. The sensor could be used as a viable staining agent and for cell growth dynamics study.
4. The use of $\alpha$-cyclodextrin enhances the solubility of the sensor in water.
5. The stained microbial cell could be seen distinctly through light microscope as well as in the culture broth with naked eyes along with the sensor.
6. The sensor is nonlipophilic in nature.
7. The staining process is easy (single staining agent), rapid and ecofriendly.
8. This is a unique staining agent that can distinguish between *Gram +ve* and *Gram -ve* bacteria in just one step.

## Claims

1. A compound of formula 1A

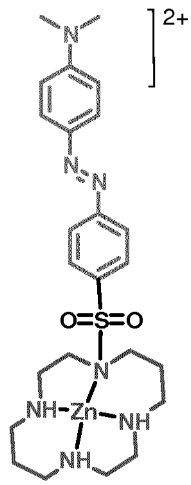

Formula 1A

2. A compound as claimed in claim 1, wherein said compound is a colorimetric sensor and useful as a viable staining agent for living cells.

3. A compound as claimed in claim 2, wherein said living cells are selected from Prokaryotic and Eukaryotic cells.

4. A compound as claimed in claim 2, wherein prokaryotic cells used are *Bacillus* species and *Pseudomonas* species.

5. A compound as claimed in claim 2, wherein Eukaryotic cells used is yeast (*Saccharomyces cerevisiae*).

6. A process for the preparation of the compound of formula **1A** comprising the steps of:

i. dissolving 1,4,8,11-tetraazacyclotetradecane in a solvent under mild stirring at the rate of 300 to 600 rpm

(rotation per minute) at temperature in the range of 25-30°C;
ii. keeping the solution as obtained in step (i) in ice at temperature in the range of 2 to 5°C;
iii. adding triethyl amine base in the solution as obtained in step (ii) at the rate of 0.5 to 1.0 mL/minute under mild stirring at the rate of 300 to 600 rpm;
iv. adding separately prepared solution of 4-(4'-dimethylamino-phenyl azo) benzene sulphonyl chloride in tetrahydrofuran in the solution as obtained in step (iii) at a rate in the range of 15 to 60 mL/hour at a temperature in the range of 0 to 5°C;
v. stirring of the resulting mixture as obtained in step (iv) in the temperature range of 25 to 30°C for a period in the range of 5 to 10 hours;
vi. refluxing the mixture as obtained in step (v) for a period in the range of 20 to 60 minutes at temperature in the range of 65 to 70°C;
vii. filtering the mixture as obtained in step (vi) to separate the precipitate of ligand;
viii. washing and drying the precipitate of ligand as obtained in step (vii);
ix. dissolving the ligand as obtained in step (viii) in alcohol at temperature in range of 20 to 25°C;
x. adding zinc nitrate solution (0.3 to 0.8 mole equivalent of the ligand) in the solution of ligand as obtained in step (ix) at a temperature in the range of 20 to 25°C;
xi. stirring the solution as obtained in step (x) at temperature in the range of 20 to 25°C for a period in the range of 12 to 24 hours;
xii. agitating the solution as obtained in step (xi) at a temperature in the range of 10 to 20°C for a period in the range of 2 to 5 hours;
xiii. filtering and washing the precipitate as obtained in step (xii) with chloroform to obtain compound of formula **1A**.

**7.** A process as claimed in step (i) of claim 6, wherein solvent used is selected from the group consisting of chloroform, methanol, dichloromethane and tetrahydrofuran (THF).

**8.** A process as claimed in step (ix) of claim 6, wherein alcohol used is selected from methanol and ethanol.

**9.** A process for staining living cells using the compound as claimed in claim 1, wherein the said steps comprising:

a cultivating the (i) *Saccharomyces* species (MTCC 5548) in glucose yeast extract agar (GYE) media, (ii) *Bacillus* species (MTCC 5549) in Nutrient broth and (iii) *Pseudomonas* species (MTCC 5550) in King's B medium at a temperature in the range of 30 to 40°C for a period in the range of 6 to 36 hours;
b incubating the cells of step (a) with $0.6x10^{-4}$ to $1.2x10^{-4}$ M compound of formula 1A in the temperature range of 28 to 38°C for a period in the range of 5 to 20 minutes;
c fixing the incubated sample as obtained in step (b) with gluteraldehyde in buffer at pH in the range of 7.4 to 7.6 for period in the range of 10 to 12 hours at a temperature in the range of 28 to 38°C;
d washing the sample as obtained in step (c) with said buffer for period in the range of 0.5 to 1.0 hour;
e post fixing the sample as obtained in step (d) with osmium tetraoxide and buffer and washing with the said buffer;
f removing water from sample as obtained in step (e) by graded water ethanol series;
g rinsing the sample as obtained in step (f) in said buffer and coating with gold in a sputter coater to obtain stained specimen for viewing under the simple microscope;
h distinguishing the living cells with different intensity of color and shape of the stained cells.

**10.** A process as claimed in claim 9, wherein reversible binding of compound of formula 1A is checked by destaining the cells with sodium citrate 20% (w/v) solution through light microscopy and UV-Vis spectroscopy.

**11.** A process as claimed in claim 9, wherein absorption spectral titration curve of compound of formula 1A shows 40 nm spectral shift after addition of ATP and binding constant of compound with ATP is (978 $\pm$ 4) $M^{-1}$.

**12.** Compound of formula **1A** as claimed in claim 1, wherein solubility of compound of formula **1A** in water is increased about 7 to 7.5 times in the presence of $\alpha$-CD (cyclodextrin).

**13.** Compound of formula **1A** as claimed in claim 1, wherein intensity of the staining of compound of formula **1A** is increased in the presence of $\alpha$-CD (cyclodextrin) by showing absorption maxima for Zn (II) based colorimetric sensor **L.Zn** tend to shift by 5 nm on addition of aqueous solution of $\alpha$-CD (cyclodextrin).

**14.** A process as claimed in claim 9, wherein SEM images of *Saccharomyces species* cell surfaces without dye were found to be smooth, while *Saccharomyces species* cell surfaces in presence of dye were found to be rough.

**Patentansprüche**

1. Verbindung der Formel 1A

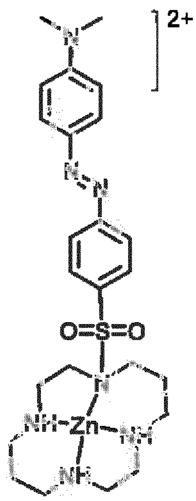

Formel 1A

2. Verbindung nach Anspruch 1, wobei besagte Verbindung ein kolorimetrischer Sensor und als ein überlebensfähiges Mittel zum Anfärben für lebende Zellen verwendbar ist.

3. Verbindung nach Anspruch 2, wobei besagte lebende Zellen ausgewählt sind aus prokaryotischen und eukaryotischen Zellen.

4. Verbindung nach Anspruch 2, wobei die verwendeten prokaryotischen Zellen *Bacillus*-Spezies und *Pseudomonas*-Spezies sind.

5. Verbindung nach Anspruch 2, wobei die verwendeten eukaryotischen Zellen Hefe (*Saccaromyces cerevisiae*) sind.

6. Verfahren zum Herstellen der Verbindung der Formel **1A**, umfassend die Schritte aus:

i. Lösen von 1,4,8,11-Tetraazacyclotetradecan in einem Lösungsmittel unter leichtem Rühren mit der Geschwindigkeit von 300 bis 600 Upm (Umdrehungen pro Minute) bei einer Temperatur in dem Bereich von 25-30°C;
ii. Halten der Lösung, welche in Schritt (i) erhalten wird, in Eis bei einer Temperatur in dem Bereich von 2 bis 5°C;
iii. Zugeben der Base Triethylamin in die Lösung, welche in Schritt (ii) erhalten wird, mit der Geschwindigkeit von 0,5 bis 1,0 ml/Minute unter leichtem Rühren mit der Geschwindigkeit von 300 bis 600 Upm;
iv. getrenntes Zugeben der hergestellten Lösung aus 4-(4'-Dimethylaminophenylazo)benzensulfonylchlorid in Tetrahydrofuran in die Lösung, welche in Schritt (iii) erhalten wird, mit einer Geschwindigkeit in dem Bereich von 15 bis 60 ml/Stunde bei einer Temperatur in dem Bereich von 0 bis 5°C;
v. Rührern der resultierenden Mischung, welche in Schritt (iv) erhalten wird, in dem Temperaturbereich von 25 bis 30°C für eine Dauer in dem Bereich von 5 bis 10 Stunden;
vi. Refluxieren der Mischung, welche in Schritt (v) erhalten wird, für eine Dauer in dem Bereich von 20 bis 60 Minuten bei einer Temperatur in dem Bereich von 65 bis 70°C;
vii. Filtrieren der Mischung, welche in Schritt (vi) erhalten wird, um den Niederschlag des Liganden zu trennen;
viii. Waschen und Trocknen des Niederschlags des Liganden, welcher in Schritt (vii) erhalten wird;
ix. Lösen des Liganden, welcher in Schritt (viii) erhalten wird, in Alkohol bei einer Temperatur in dem Bereich von 20 bis 25°C;
x. Zugeben von Zinknitratlösung (0,3 bis 0,8 Moläquivalente des Liganden) in die Lösung des Liganden, welcher in Schritt (ix) erhalten wird, bei einer Temperatur in dem Bereich von 20 bis 25°C;
xi. Rühren der Lösung, welche in Schritt (x) erhalten wird, bei einer Temperatur in dem Bereich von 20 bis 25°C für eine Dauer in dem Bereich von 12 bis 24 Stunden;
xii. Agitieren der Lösung, welche in Schritt (xi) erhalten wird, bei einer Temperatur in dem Bereich von 10 bis

20°C für eine Dauer in dem Bereich von 2 bis 5 Stunden;
xiii. Filtrieren und Waschen des Niederschlags, welcher in Schritt (xii) erhalten wird, mit Chloroform, um eine Verbindung der Formel **1A** zu erhalten.

**7.** Verfahren wie in Schritt (i) von Anspruch 6, wobei das verwendete Lösungsmittel ausgewählt ist aus der Gruppe bestehend aus Chloroform, Methanol, Dichlormethan und Tetrahydrofuran (THF).

**8.** Verfahren wie in Schritt (ix) von Anspruch 6, wobei der verwendete Alkohol ausgewählt ist aus Methanol und Ethanol.

**9.** Verfahren zum Anfärben von lebenden Zellen unter Verwendung der Verbindung nach Anspruch 1, wobei die besagten Schritte umfassen:

a Kultivieren der (i) *Saccaromyces*-Spezies (MTCC 5548) in einem Agarmedium aus Glucose-Hefeextrakt (GYE), (ii) *Bacillus*-Spezies (MTCC 5549) in Nährlösung und (iii) *Pseudomonas*-Spezies (MTCC 5550) in King's B-Medium bei einer Temperatur in dem Bereich von 30 bis 40°C für eine Dauer in dem Bereich von 6 bis 36 Stunden;
b Inkubieren der Zellen aus Schritt (a) mit 0,6 x $10^{-4}$ bis 1,2 x $10^{-4}$ M der Verbindung der Formel 1A in dem Temperaturbereich von 28 bis 38°C für eine Dauer in dem Bereich von 5 bis 20 Minuten;
c Fixieren der inkubierten Probe, welche in Schritt (b) erhalten wird, mit Gluteraldehyd in Puffer bei einem pH in dem Bereich von 7,4 bis 7,6 für eine Dauer in dem Bereich von 10 bis 12 Stunden bei einer Temperatur in dem Bereich von 28 bis 38°C;
d Waschen der Probe, welche in Schritt (c) erhalten wird, mit besagtem Puffer für eine Dauer in dem Bereich von 0,5 bis 1,0 Stunde;
e Nachträgliches Fixieren der Probe, welche in Schritt (d) erhalten wird, mit Osmiumtetraoxid und Puffer und Waschen mit dem besagten Puffer;
f Entfernen von Wasser aus der Probe, welche in Schritt (e) erhalten wird, durch eine abgestufte Wasser-Ethanol-Folge;
g Spülen der Probe, welche in Schritt (f) erhalten wird, in besagtem Puffer und Beschichten mit Gold in einer Sputter-Beschichtungsanlage, um angefärbte Proben zum Betrachten unter dem einfachen Mikroskop zu erhalten;
h Unterscheiden der lebenden Zellen mit unterschiedlicher Intensität von Farbe und Form der angefärbten Zellen.

**10.** Verfahren nach Anspruch 9, wobei das reversible Binden der Verbindung der Formel **1A** durch Entfärben der Zellen mit einer 20%igen (w/v) Lösung aus Natriumcitrat durch Lichtmikroskopie und UV-Vis-Spektroskopie geprüft wird.

**11.** Verfahren nach Anspruch 9, wobei die Absorptionsspektraltitrationskurve der Verbindung der Formel **1A** eine Spektralverschiebung von 40 nm nach Zugabe von ATP zeigt und die Bindungskonstante der Verbindung mit ATP (978$\pm$4) $M^{-1}$ ist.

**12.** Verbindung der Formel **1A** nach Anspruch 1, wobei die Löslichkeit der Verbindung der Formel **1A** in Wasser in der Anwesenheit von $\alpha$-CD (Cyclodextrin) etwa 7- bis 7,5-mal erhöht ist.

**13.** Verbindung der Formel **1A** nach Anspruch 1, wobei die Intensität des Anfärbens der Verbindung der Formel **1A** in der Anwesenheit von $\alpha$-CD (Cyclodextrin) durch Zeigen eines Absorptionsmaximums für den Zn(II)-basierten kolorimekrischen Sensor **L.Zn**, aufgrund der Verschiebung von 5 nm nach Zugabe von wässriger Lösung von $\alpha$-CD (Cyclodextrin), erhöht ist.

**14.** Verfahren nach Anspruch 9, wobei sich die SEM-Aufnahmen von Zelloberflächen der *Saccaromyces*-Spezies ohne Farbstoff als eben erwiesen, während sich die Zelloberflächen der *Saccharomyces*-Spezies in Anwesenheit von Farbstoff als rau erwiesen.

## Revendications

**1.** Composé de la formule 1A

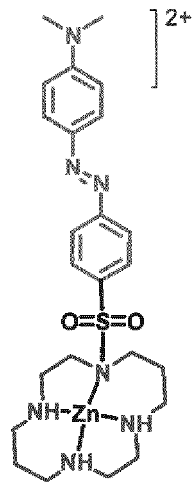

Formule 1A

2. Composé selon la revendication 1, lequel composé est un détecteur colorimétrique qui est utile en tant qu'agent de coloration viable pour les cellules vivantes.

3. Composé selon la revendication 2, dans lequel lesdites cellules vivantes sont sélectionnées parmi des cellules procaryotes et eucaryotes.

4. Composé selon la revendication 2, dans lequel les cellules procaryotes utilisées sont les espèces *Bacillus* et les espèces *Pseudomonas*.

5. Composé selon la revendication 2, dans lequel les cellules eucaryotes utilisées sont celles de la levure (*Saccharomyces cerevisiae*).

6. Procédé de préparation du composé de la formule **1A** comprenant les étapes consistant à :

i. dissoudre du 1,4,8,11-tétraazacyclotétradécane dans un solvant sous agitation légère à une vitesse de 300 à 600 rpm (rotations par minute) à une température d'environ 25 à 30°C ;
ii. conserver la solution obtenue à l'étape (i) dans de la glace à une température d'environ 2 à 5°C ;
iii. ajouter une base de triéthylamine dans la solution obtenue à l'étape (ii) à un débit de 0,5 à 1,0 ml/minute sous agitation légère à une vitesse de 300 à 600 rpm ;
iv. ajouter séparément une solution préparées de chlorure de sulphonyle 4-(4'-diméthylamino-phényl azo) benzène dans du tétrahydrofurane à la solution obtenue à l'étape (iii) à un débit de 15 à 60 ml/heure à une température d'environ 0 à 5°C ;
v. mélanger le mélange résultant obtenu à l'étape (iv) à une température d'environ 25 à 30°C pendant une durée allant de 5 à 10 heures ;
vi. mettre à ébullition le mélange obtenu à l'étape (v) pendant une durée allant de 20 à 60 minutes à une température d'environ 65 à 70°C ;
vii. filtrer le mélange obtenu à l'étape (vi) pour séparer le précipité de ligand ;
viii. laver et sécher le précipité de ligand obtenu à l'étape (vii);
ix. dissoudre le ligand obtenu à l'étape (viii) dans de l'alcool à une température d'environ 20 à 25°C ;
x. ajouter une solution de nitrate de zinc (0,3 à 0,8 équivalent molaire du ligand) à la solution de ligand obtenue à l'étape (ix) à une température d'environ 20 à 25°C ;
xi. mélanger la solution obtenue à l'étape (x) à une température d'environ 20 à 25°C pendant une durée allant de 12 à 24 heures ;
xii. agiter la solution obtenue à l'étape (xi) à une température d'environ 10 à 20°C pendant une durée allant de 2 à 5 heures ;
xiii. filtrer et laver le précipité obtenu à l'étape (xii) avec du chloroforme pour obtenir le composé de la formule **1A**.

7. Procédé selon le mode de réalisation de l'étape (i) de la revendication 6, dans lequel le solvant utilisé est sélectionné parmi le groupe constitué de chloroforme, de méthanol, de dichlorométhane et de tétrahydrofurane (THF).

**8.** Procédé selon le mode de réalisation de l'étape (ix) de la revendication 6, dans lequel l'alcool utilisé est sélectionné parmi le méthanol et l'éthanol.

**9.** Procédé de coloration de cellules vivantes en utilisant le composé selon la revendication 1, comprenant les étapes consistant à :

a cultiver (i) les espèces *Saccharomyces* (MTCC 5548) dans un milieu gélosé d'extrait de levure de glucose (GYE), (ii) les espèces *Baciilus* (MTCC 5549) dans un milieu nutritif et (iii) les espèces *Pseudomonas* (MTCC 5550) dans un milieu B de King à une température d'environ 30 à 40°C pendant une durée allant de 6 à 36 heures ;
b incuber les cellules de l'étape (a) avec 0,6 x $10^{-4}$ à 1,2 x $10^{-4}$ M du composé de la formule 1A à une température d'environ 28 à 38°C pendant une durée allant de 5 à 20 minutes ;
c fixer l'échantillon incubé obtenu à l'étape (b) avec du glutéraldéhyde dans un tampon à un pH compris entre 7,4 à 7,6 pendant une durée allant de 10 à 12 heures à une température d'environ 28 à 38°C ;
d laver l'échantillon obtenu à l'étape (c) avec ledit tampon pendant une durée allant de 0,5 à 1,0 heure ;
e post-fixer l'échantillon obtenu à l'étape (d) avec du tétroxyde d'osmium et le tampon et le laver avec ledit tampon ;
f enlever l'eau de l'échantillon obtenu à l'étape (e) avec des volumes calibrés d'éthanol dans de l'eau ;
g rincer l'échantillon obtenu à l'étape (f) dans ledit tampon et le pulvériser cathodiquement d'or pour obtenir un spécimen coloré à examiner au simple microscope ;
h distinguer les cellules vivantes ayant une intensité de couleur et une forme différentes des cellules colorées.

**10.** Procédé selon la revendication 9, dans lequel la liaison réversible du composé de la formule 1A est vérifiée en décolorant les cellules avec une solution de citrate de sodium à 20% (p/v) par microscope optique et spectroscopie dans l'ultraviolet et dans le visible (UV-Vis).

**11.** Procédé selon la revendication 9, dans lequel la courbe de titrage spectral de l'absorption du composé de la formule 1A montre un décalage spectral de 40 nm après l'ajout d'adénosine triphosphate (ATP) et la constante de liaison du composé avec l'ATP est de (978 $\pm$ 4) $M^{-1}$.

**12.** Composé de la formule **1A** selon la revendication 1, **caractérisé par le fait que** la solubilité du composé de la formule **1A** dans l'eau augmente d'environ 7 à 7,5 fois en présence de $\alpha$-CD (cyclodextrine).

**13.** Composé de la formule **1A** selon la revendication 1, dans lequel l'intensité de la coloration du composé de la formule **1A** est plus élevée en présence de $\alpha$-CD (cyclodextrine) en affichant des maximums d'absorption avec le capteur colorimétrique à base de Zn (II). **L.Zn** tend à dériver de 5 nm lors de l'ajout d'une solution aqueuse de $\alpha$-CD (cyclodextrine).

**14.** Procédé selon la revendication 9, dans lequel les cellules des espèces *Saccharomyces* sans colorant se sont avérées être lisses, tandis que les surfaces de cellules des espèces *Saccharomyces* en présence de colorant se sont révélées rugueuses sur les images SEM.

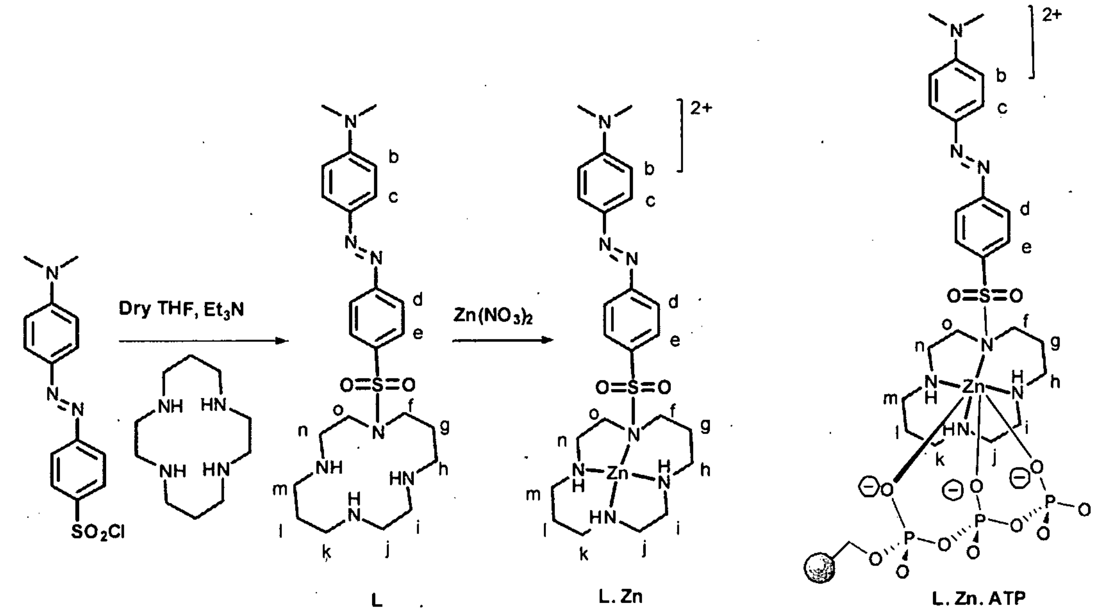


L (Formula 2) L. Zn (Formula 1A) L. Zn. ATP (Formula 1B)

**Scheme1**

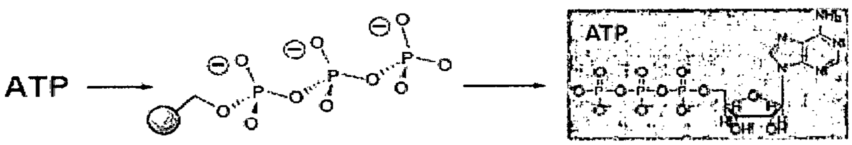


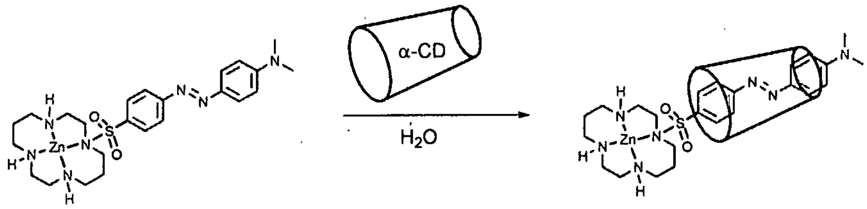


L.Zn L.Zn.CD

**Scheme 2**

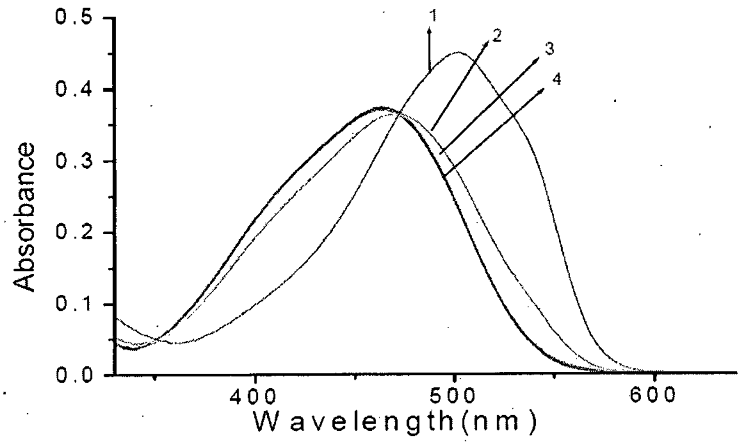


Figure 1

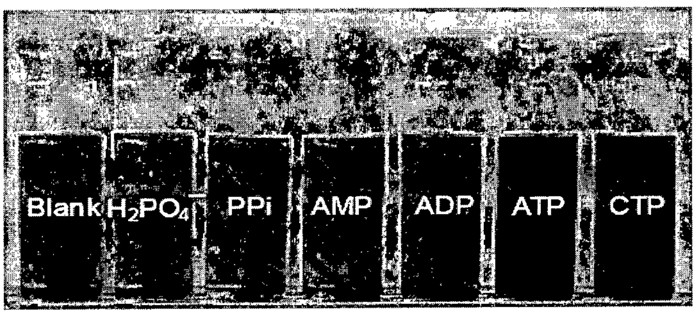


Figure 2

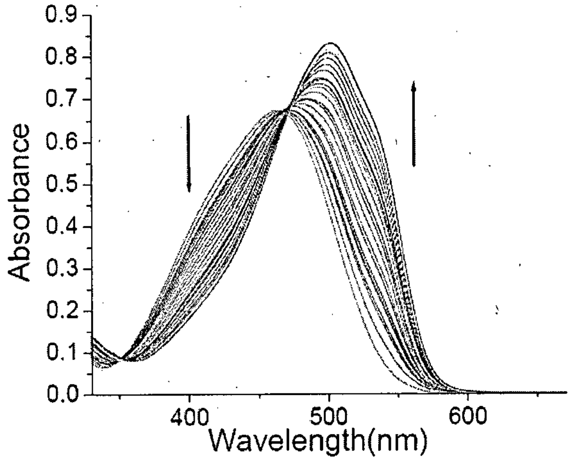


Figure 3

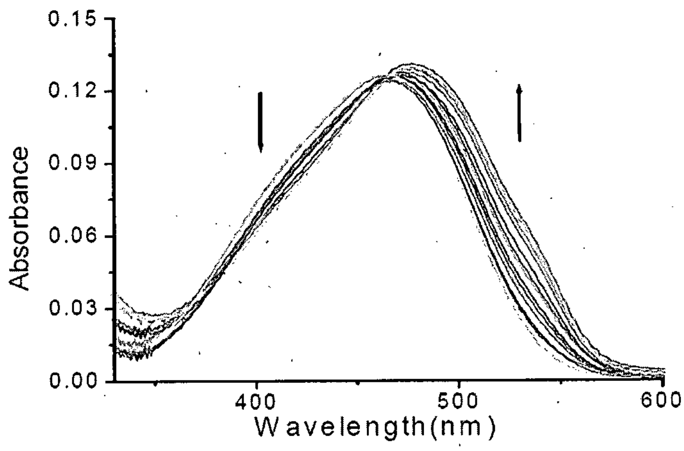


Figure 4

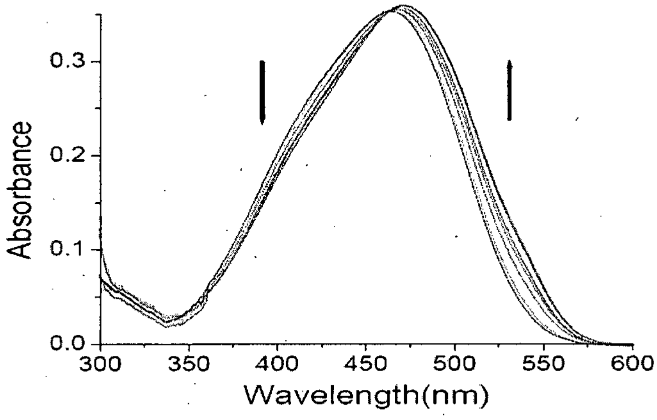


Figure 5

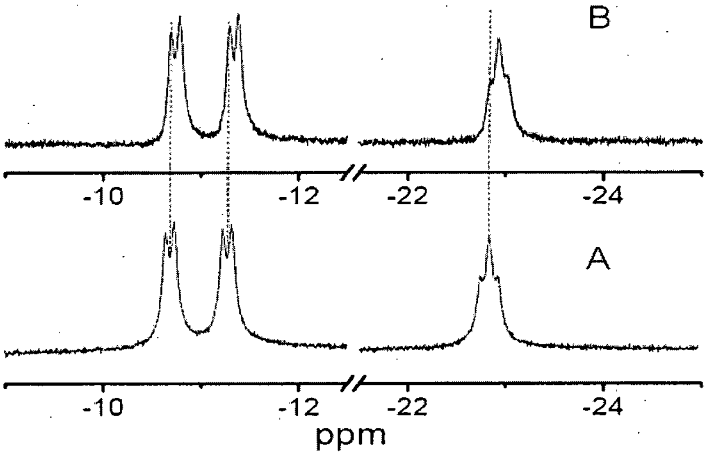


Figure 6 A

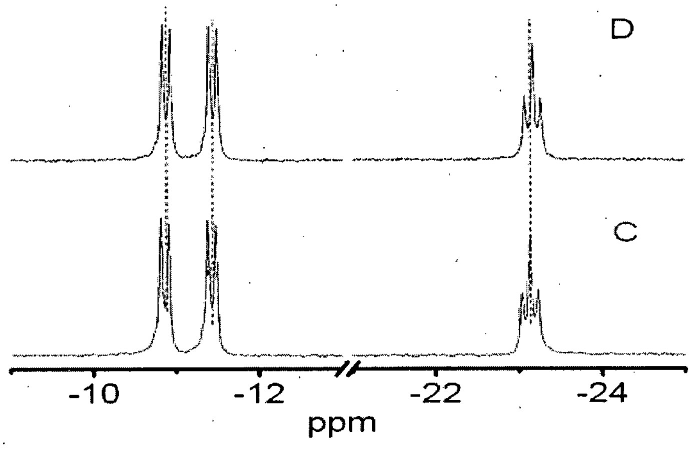


Figure 6B

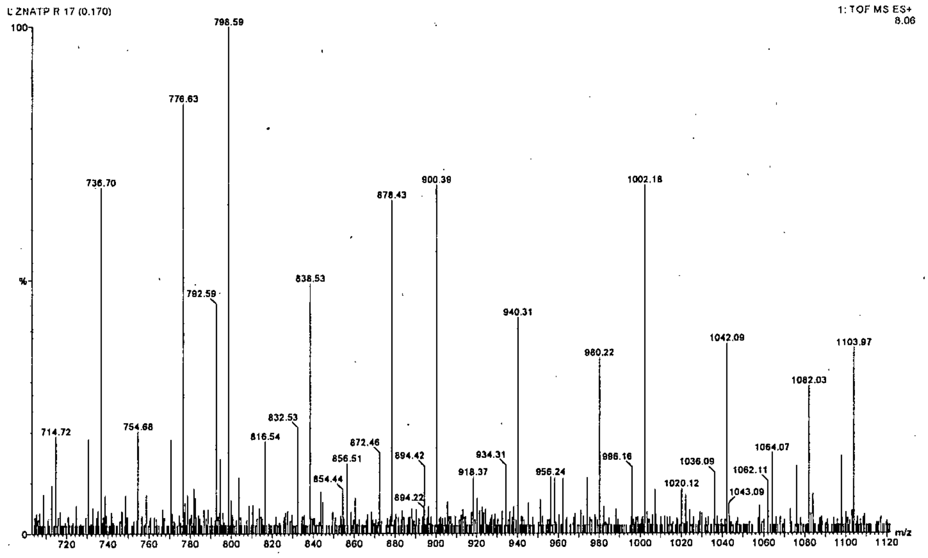


Figure 7

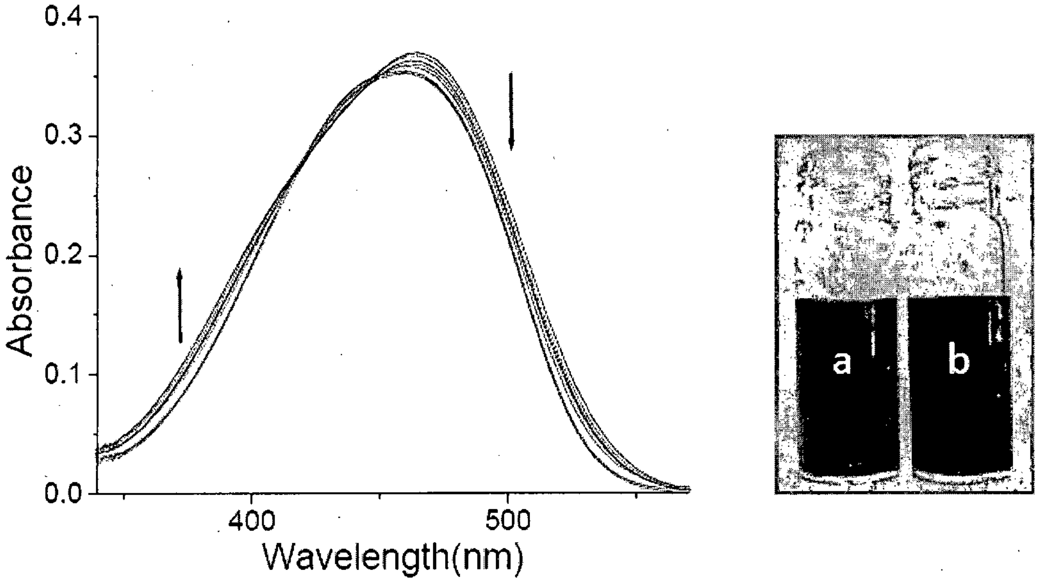


Figure 8

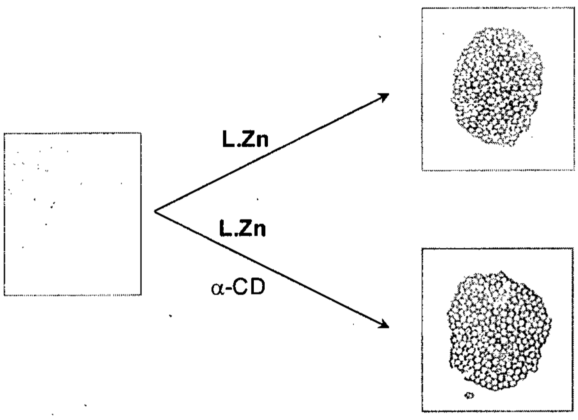


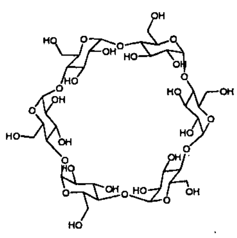

α-CD

Figure 9A

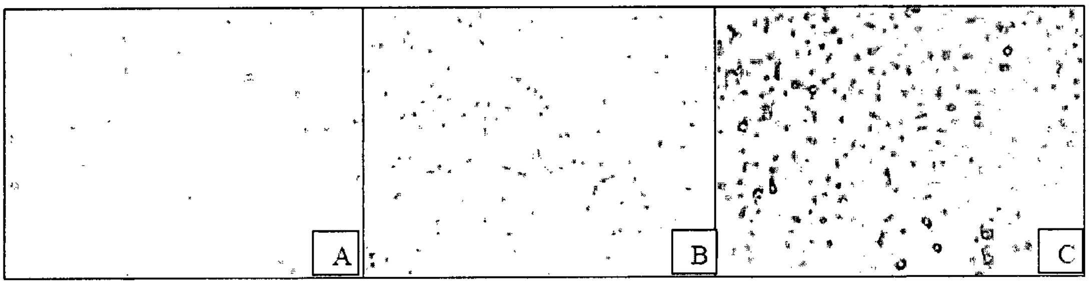


Figure 9B

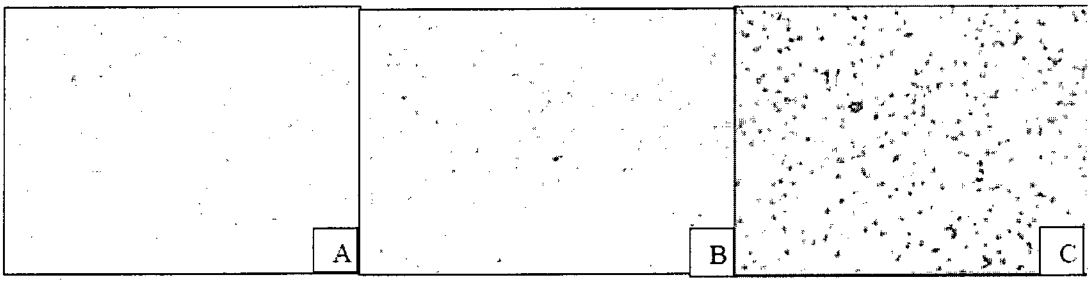


Figure 9C

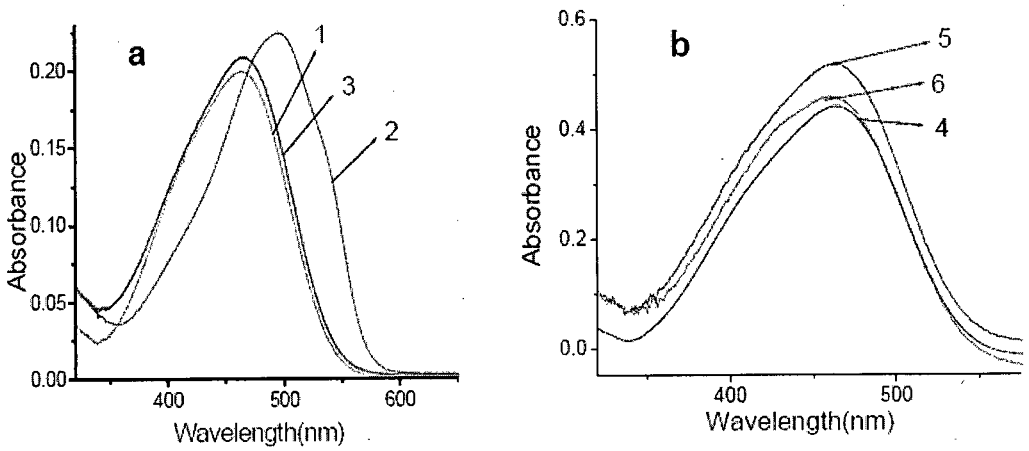


Figure 10A

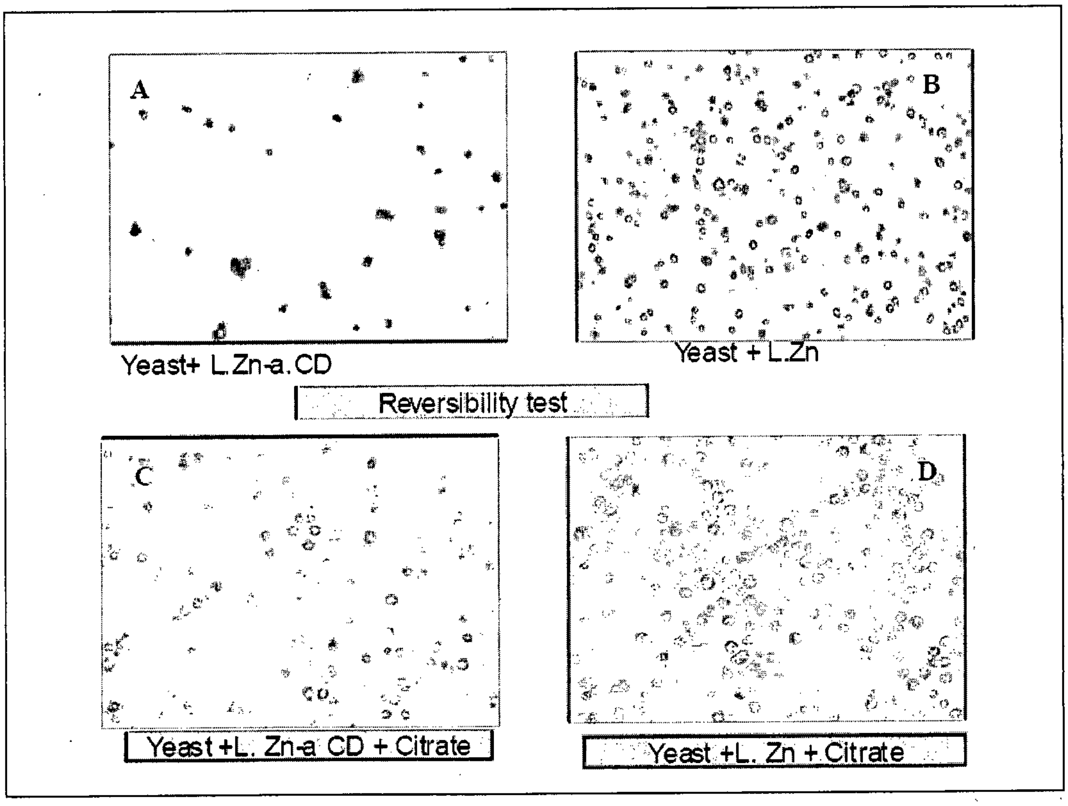


Figure 10B

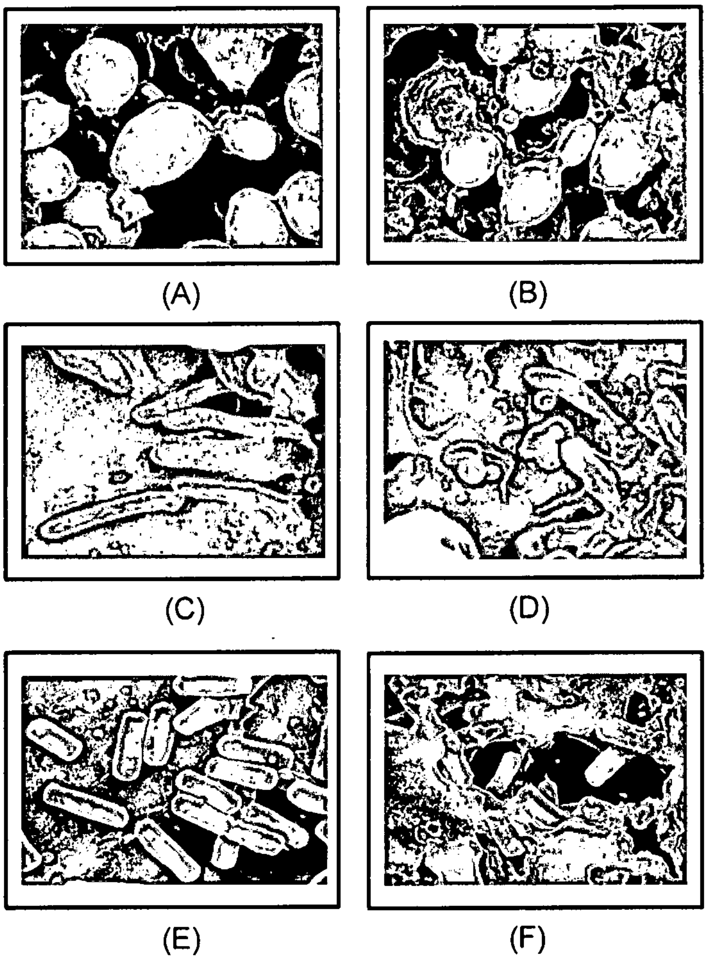

Figure 11

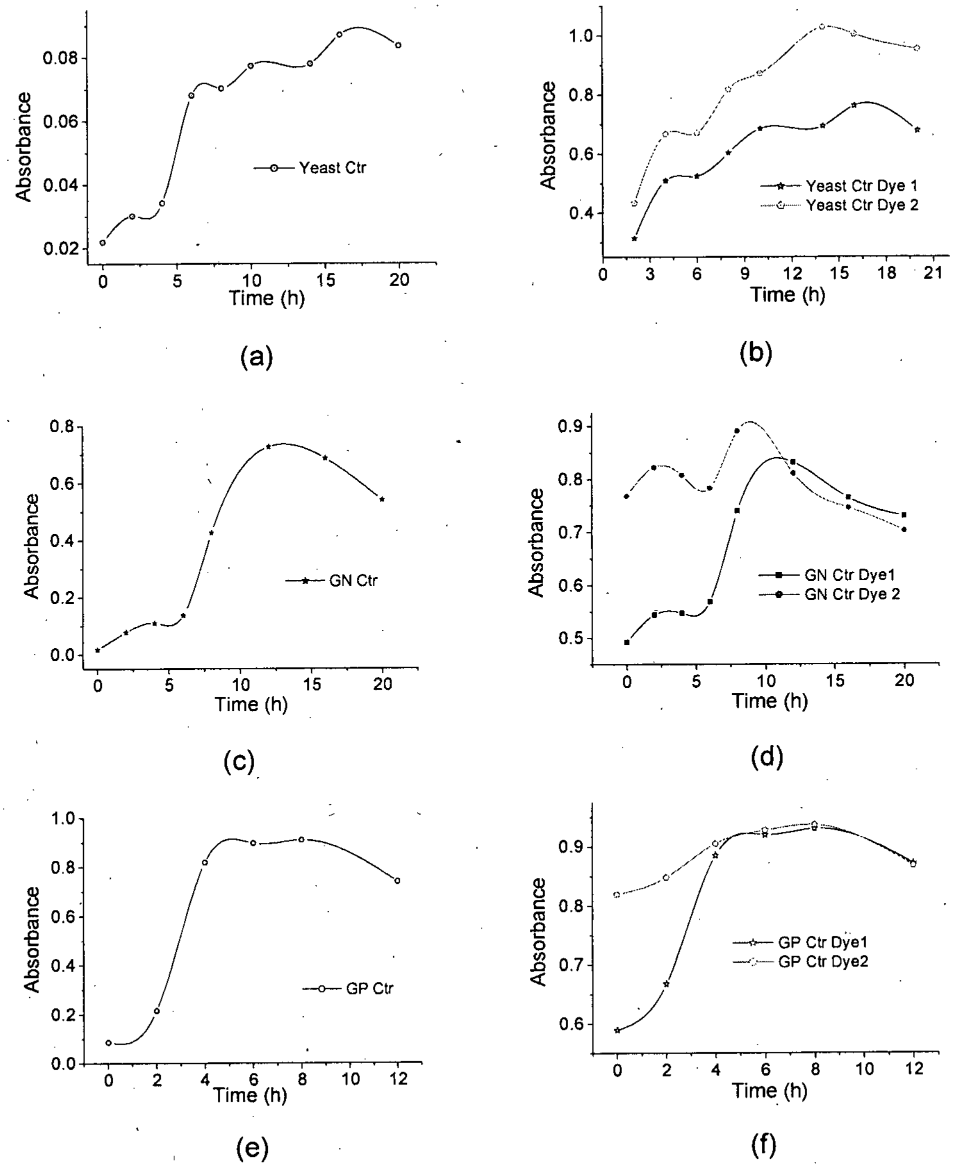
Absorbance
Time (h)
Yeast Ctr
(a)
Absorbance
Time (h)
Yeast Ctr Dye 1
Yeast Ctr Dye 2
(b)
Absorbance
Time (h)
GN Ctr
(c)
Absorbance
Time (h)
GN Ctr Dye1
GN Ctr Dye 2
(d)
Absorbance
Time (h)
GP Ctr
(e)
Absorbance
Time (h)
GP Ctr Dye1
GP Ctr Dye2
(f)

**Figure 12**

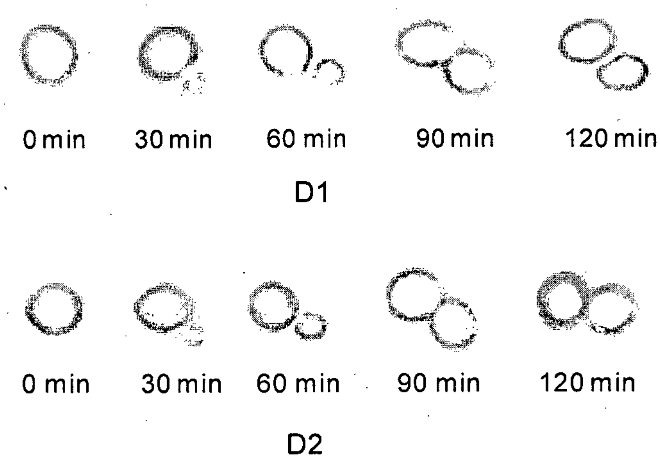
0 min
30 min
60 min
90 min
120 min
D1
0 min
30 min
60 min
90 min
120 min
D2

**Figure 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VÁZQUEZ, M. ; FABBRIZZI, L. ; TAGLIETTI A. ; PEDRIDO, R. M. ; GONZÁLEZ-NOYA, A. M. ; BERMEJO, M. R.** *Angew. Chem. Int. Ed.,* 2004, vol. 43, 1962
- **LIN, Z. ; OU, S. ; DUAN, C. ; ZHANG, B. ; BAI, Z.** *Chem. Commun.,* 2006, 624
- **MIYAJI, H. ; SESSLER J. L.** *Angew. Chem. Int. Ed.,* 2001, vol. 40, 154
- **LIBRA, E. R. ; SCOTT, M. J.** *Chem. Commun.,* 2006, 1485
- **ZHANG, D. ; ZHANG, M. ; LIU Z. ; YU, M. ; LI, F. ; YI, T. ; HUANG, C.** *Tetrahedron Lett.,* 2006, vol. 47, 7093
- **HAN, M. S. ; KIM, D.H.** *Angew. Chem. Int. Ed.,* 2002, vol. 41, 3809
- **JOSE, D. A. ; KAR, P. ; KOLEY, D. ; GANGULY, B. ; THIEL, W. ; GHOSH, H.N. ; DAS, A.** *Inorg. Chem.,* 2007, vol. 46, 5576
- **JOSE, D. A. ; KUMAR, D. K. ; KAR, P. ; VERMA, S. ; GHOSH, A. ; GANGULY, B. ; GHOSH, H. N. ; DAS, A.** *Tetrahedron,* 2007, vol. 63, 12007
- **JOSE, D. A. ; KUMAR, D. K. ; GANGULY, B. ; DAS, A.** *Org. Lett.,* 2004, vol. 6, 3445
- **JOSE, D. A. ; KUMAR, D. K. ; GANGULY, B. ; DAS, A.** *Tetrahedron Lett.,* 2005, vol. 46, 5343
- **JOSE,D. A. ; SINGH, A. ; GANGULY, B. ; DAS, A.** *Tetrahedron Lett.,* 2007, vol. 48, 3695
- **HU, S. ; GUO, Y. ; XU, J. ; SHAO, S.** *Org. Biomol. Chem.,* 2008, vol. 6, 2071
- **HU, S. ; GUO, Y. ; XUA, J. ; SHAO, S.** *Org. Biomol. Chem.,* 2008, vol. 6, 2071
- **ZEIDER, F. H.** *Inorg. Chem.,* 2008, vol. 47, 1264
- **KHAKH, B. S. ; NORTH, R.A.** *Nature,* 2006, vol. 442, 527
- **GHOSH, A. ; SHRIVASTAV, A. ; JOSE, D. A. ; MISHRA, S. K. ; MISHRA, S. ; DAS, A.** *Anal. Chem.,* 2008, vol. 80, 5312
- **JOSE, D. A. ; MISHRA. S. ; GHOSH, A. ; SHRIVASTAV, A. ; MISHRA, S. K. ; DAS, A.** *Org. Lett.,* 2007, vol. 9, 1979
- **IVANOVA, E. P. ; ALEXEEVA, Y. V. ; PHAM, D. K. ; WRIGHT, J. P. ; NICOLAU, D. V.** *Int. Microbiol.,* 2006, vol. 9, 37
- **LEEVY, W. M. ; JOHNSON, J. R. ; LAKSHMI, C. ; MORRIS, J. ; MARQUEZ, M. ; SMITH, B. D.** *Chem. Commun.,* 2006, 1595
- **GORDON, J. L.** *Biochem. J.,* 1986, vol. 233, 309
- **NELSON, D.L. ; COX, M. M.** Lehninger: Principles of Biochemistry. Worth Publishers, 2003
- **BERGEY, D. H. ; JOHN, G. H. ; NOEL, R. K. ; PETER, H. A. S.** Bergey's Manual of Determinative Bacteriology. Lippincott Williams & Wilkins, 1994
- **CAHILL, G. ; WALSH, P.K. ; DONNELLY, D.** *J. Am. Soc. Brew. Chem.,* 1999, vol. 57, 72
- **ZHANG, T. ; FANG, H. H. P.** *Biotechnol. Lett.,* 2004, vol. 26, 989
- **BISWAS, K. ; RIEGER, K. ; MORSCHHÄUSER.** *J. Gene,* 2003, vol. 307, 151
- **DAN, N. P. ; VISVANATHAN, C. ; BASU, B.** *Bioresour. Technol.,* 2003, vol. 87, 51
- **MILLSAP, K.W. ; M. VAN DER, H. C. ; BOS, R. ; BUSSCHER, H. J.** *FEMS, Microbial. Rev.,* 1998, vol. 21, 321
- **NARVHUS, J. A. ; GADAGA, T. H.** *Int. J. Food Microbiol.,* 2003, vol. 86, 51S
- **BASKIN, D.S. ; NGO, H. ; DIDENKO, V.V.** *Toxicol. Sci.,* 2003, vol. 74, 361
- **SHAWN C. MCCLESKEY et al.** *J. Am. Chem. Soc.,* 2003, vol. 125, 1114
- **CHUN LI et al.** *Angew. Chem. Int. Ed.,* 2005, vol. 44, 6371
- **SHENLIANG WANG et al.** *J. Am. Chem. Soc.,* 2006, vol. 128, 10380
- **OKOH et al.** *Biochemistry,* 2006, vol. 45, 14764
- **AKIO OJIDA et al.** *Chem. Asian J.,* 2006, vol. 1, 555
- **AKIO OJIDA et al.** *Angew. Chem. Int. Ed.,* 2006, vol. 45, 5518
- **ZDENEK KEJIK et al.** *Chem. Commun.,* 2006, 1533
- **YASUYUKI TSUBOI et al.** *Applied Surface Science,* 2007, vol. 253, 8422
- **DAS et al.** *Org. Lett.,* 2007, vol. 9, 1979
- *Anal. Chem.,* 2008, vol. 80, 5312
- **PENG JIANG et al.** *Biochemistry,* 2007, vol. 46, 12979
- **HAO WANG et al.** *Org. Biomol. Chem.,* 2008, vol. 6, 162
- **TAE-HYUK KWON.** *Chem. Eur. J.,* 2008, vol. 14, 9613
- **AKIHIKO ISHIDA et al.** *Anal Bioanal Chem,* 2008, vol. 392, 987
- **YIMIN SUN et al.** *Org. Biomol. Chem.,* 2008, vol. 6, 3044

- **ITARU HAMACHI et al.** *J. Am. Chem. Soc.,* 2008, vol. 130, 12095
- **HONGMEI WU et al.** *Inorg. Chem.,* 2009, vol. 48, 408
- **AIKO NONAKA et al.** *Org. Biomol. Chem.,* 2008, vol. 6, 3621
- *Organic Letters,* 2007, vol. 9 (10), 1979-1982
- *Anal. Chem.,* 2008, vol. 80, 5312-5319
- **NELSON, D. L. ; COX, M. M.** Lehninger: Principles of Biochemistry. WorthPublishers, 2003
- **JOHN, D. H. ; NOEL, G. H. ; PETER, R. K. ; BERGEY'S, H. A. S.** Manual of Determinative Bacteriology. Lippincott Williams & Wilkins, 1994
- **WEXLER, H. M.** *Clinical Infectious Diseases,* 1997, vol. 25, 284
- **IVANOVA, E. P. ; ALEXEEVA, Y. V. ; PHAM, D. K. ; NICOLAU, W. D.** *Internationalmicrobiology,* 2006, vol. 9, 37
- **WERNER, T. P. ; AMRHEIN, N. ; FREIMOSER, F. M.** *BMC Plant Biology,* 2007, vol. 7, 51
- **SHUKLA, A. D. ; BAJAJ, H. C. ; DAS, A.** *Angew. Chem. Int. Ed.,* 2001, vol. 40, 446
- **SHUKLA, A. D. ; BAJAJ, H. C. ; DAS, A.** *Nat. Acad. Science,* vol. 114, 431
- **BEER, A.J. ; MACARTNEY, D. H.** *Inorg. Chem.,* 2000, vol. 39, 1410
- **MACARTNEY, D.H. ; WADDLING, C. A.** *Inorg. Chem.,* 1994, vol. 33, 5912
- **PHILIP, D. ; STODDART, J.F.** *Angew. Chem. Int. Ed.,* 1996, vol. 35, 1154
- **OJIDA, A. ; PARK, S. K. ; MITO-OKA, Y. ; HAMACHI, I.** *Tetrahedron Lett.,* 2002, vol. 43, 6193
- **LINDLOY, L. F. ; BUSCH, D. H.** *Inorg. Chem.,* 1974, vol. 13, 2494
- **WESTER, D. ; PALENIK, G.** *J. J. Am. Chem. Soc.,* 1974, vol. 95, 6505
- **KWON, J. Y. ; JANG. Y. J. ; LEE, Y. J. ; KIM, K. M. ; SEO M. S. ; NAM, W. ; YOON, J.** *J. Am. Chem. Soc.,* 2005, vol. 127, 10107